(19) **Europäisches Patentamt**
**European Patent Office**
**Office européen des brevets**

(11) **EP 4 692 801 A1**

(12) **EUROPEAN PATENT APPLICATION**

(43) Date of publication:
**11.02.2026 Bulletin 2026/07**

(51) International Patent Classification (IPC):
**G01N 33/574** (2006.01)

(21) Application number: **24193243.3**

(52) Cooperative Patent Classification (CPC):
**G01N 33/57423**

(22) Date of filing: **07.08.2024**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **Heckel, Tobias**
**81379 München (DE)**

• **Kauppila, Timo**
**50823 Köln (DE)**
• **Zacherle, Tobias**
**50668 Köln (DE)**
• **Hölzer, Philipp**
**Tokyo, 141-0021 (JP)**
• **Klatt, Torbjörn**
**50374 Erftstadt (DE)**

(74) Representative: **Siemens Healthineers Patent Attorneys**
**Postfach 22 16 34**
**80506 München (DE)**

(54) **MULTI-ANALYTE BLOOD TESTS TAILORED TO SEEK, ASSESS, AND SUBTYPE PULMONARY NODULES**

(57) The present invention relates to compositions for detecting a lung cancer disease, comprising peptide affinity ligands for detecting different combinations of proteins of different groups of biomarkers which include CYFRA 21-1, Prolactin, Squamous Cell Carcinoma Antigen (SCC), Her2/Neu, Cancer antigen 125 (CA125), carcinoembryonic antigen (CEA), C-reactive protein (CRP), Neuron Specific Enolase (NSE), C4 complement (C4), Interleukin 6 (IL-6), and Metallopeptidase Inhibitor 1 (TIMP-1). The invention further relates to methods of detecting or subtyping lung diseases, as well as to computer-implemented methods based on machine learning methods using expression data of the biomarkers.

FIG 1

EP 4 692 801 A1

**Description**

TECHNICAL FIELD

The present invention relates to a composition for detecting a lung cancer disease, comprising a peptide affinity ligand for the protein(s) of a group of biomarkers comprising at least CYFRA 21-1, Prolactin, Squamous Cell Carcinoma Antigen (SCC) and Her2/Neu, preferably additionally comprising a peptide affinity ligand for the protein (s) of one or more of Cancer antigen 125 (CA125), carcinoembryonic antigen (CEA), C-reactive protein (CRP), Neuron Specific Enolase (NSE) and C4 complement (C4). Also encompassed is a composition for discriminating a non-cancerous lung disease showing benign pulmonary nodules from a lung cancer disease showing cancerous lung nodules, comprising a peptide affinity ligand for the protein(s) of a group of biomarkers comprising CYFRA 21-1, CEA and Interleukin 6 (IL-6), as well as a composition for discriminating lung cancer subtypes Non-small-cell lung cancer (NSCLC) and small-cell lung cancer (SCLC), comprising a peptide affinity ligand for the protein(s) of a group of biomarkers comprising at least CYFRA 21-1, SCC, NSE, Metallopeptidase Inhibitor 1 (TIMP-1) and Her2/Neu. The invention further relates to corresponding methods of detecting the mentioned lung diseases, as well as computer-implemented methods for the analysis of a sample based on machine learning methods using expression data of the biomarkers.

BACKGROUND

A patient's journey for the early detection of lung cancer is a sequence of events that typically starts with the eligibility and the acceptance of the individual to undergo CT scans of the chest. In case the CT scan is devoid of any malignant finding, the journey continues with periodical follow-up CT scans of the lung. In case the CT scan is inconclusive with unclear findings on imaging, there is a need for additional non-invasive or minimally invasive diagnostic tests prior to transbronchial or transthoracic biopsy or diagnostic surgery such as video-assisted thoracoscopic surgery (VATS). With a secured diagnosis of lung cancer, the patient journey continues to treatment tailored to the disease stage and fitness of the patient.

Eligibility for CT scans of the lung as part of a screening program depends, for example in the USA, on dichotomous screening criteria of the U.S. Preventive Services Task Force (USPSTF). Since 2021, the USPSTF recommends low dose CT-scans for lung cancer screening in ever-smokers aged 50 to 80 years who have a 20 pack-year smoking history and currently smoke or have quit within the past 15 years. However, there are still lung cancers that will not be found using the current screening guidelines. An alternative to the USPSTF screening criteria would be criteria personalized to the individual, ideally quantitative, blood-based biomarkers adjusted to the stage of the patient journey that indicate the presence of lung cancer or a risk of developing lung cancer within a short time frame of e.g. 1 to 3 years.

Once a CT image of the chest has been acquired, most of the findings in CT images are non-cancerous findings. To reduce false positive findings for both low-dose CT screening and incidentally discovered pulmonary nodules, it is current clinical practice to use an image-based malignancy score or a risk score like the Brock score that adds clinical data such as age, gender, or family history of cancer to the radiographic characteristics. However, since the overwhelming majority of detected pulmonary nodules (80-95%) are benign, the estimated risk score is frequently incorrect.

Additional methods such as quantitative, blood-based biomarkers tailored to distinguish benign findings from early-stage cancer adjunct to imaging have been suggested to improve the risk assessment of indeterminate pulmonary nodules. However, single blood-based biomarkers failed since they are susceptible to interference by benign conditions (false-positives), or incomprehensively detect tumor heterogeneity, or they are not sensitive enough (Diamandis, 2012, BMC Med, 10 87; Yang et al., 2021, Sci Rep, 11(1), 19044).

Multi-analyte blood-based biomarkers have been shown to improve the diagnostic performance, but the usage of standard cut-off values or trade-offs between sensitivity and specificity to determine optimal performance resulted in a rate of misdiagnosis that is still unacceptably high for clinical use (Visser et al., 2023, Lung Cancer, 178, 28-36). Thus, a major challenge in the assessment of indeterminate pulmonary nodules remains the identification of better biomarker panels that can be used in clinical routine in a timely and cost-effective manner with high sensitivity and specificity.

Further, when a pulmonary nodule with high malignancy risk has been identified, definitive diagnosis including the lung cancer subtype information is required prompting an invasive procedure to get a tissue sample by transbronchial or transthoracic biopsy or diagnostic surgery such as video-assisted thoracoscopic surgery (VATS). Examining the tissue allows the discrimination between the more slowly growing Non-Small Cell Lung Cancer (NSCLC) and the aggressively and rapidly growing Small Cell Lung Cancer (SCLC). Unfortunately, these biopsies are not always adequate for histological profiling, necessitating repeated biopsies. Moreover, obtaining tissue via invasive procedures may be challenging or impossible in fragile patients or patients with small or unreachable tumors (Visser et al., 2023, Lung Cancer, 178, 28-36). Therefore, minimally invasive procedures like blood-based tumor marker tests are required to support the histological subtyping in patients for whom tissue examination is impossible or incomplete.

In summary, there are clear needs to improve the detection of lung cancer in particular through minimal-invasive tests.

SUMMARY

The present invention addresses these needs and provides in a first aspect a composition for detecting a lung cancer disease, the composition comprising a peptide affinity ligand for a protein of each biomarker of a group of biomarkers, the group of biomarkers comprising at least CYFRA 21-1, Prolactin, Squamous Cell Carcinoma Antigen (SCC) and Her2/Neu. The group may optionally, in certain embodiments, further comprise a peptide affinity ligand for a protein of one or more of Cancer antigen 125 (CA125), carcinoembryonic antigen (CEA), C-reactive protein (CRP), Neuron Specific Enolase (NSE) and C4 complement (C4).

In a further aspect, the present invention relates to a composition for discriminating a non-cancerous lung disease showing benign pulmonary nodules from a lung cancer disease showing cancerous lung nodules, the composition comprising a peptide affinity ligand for a protein of each biomarker of a group of biomarkers, the group of biomarkers comprising CYFRA 21-1, CEA and Interleukin 6 (IL-6).

In yet another aspect, the present invention relates to a composition for discriminating lung cancer subtypes Non-small-cell lung cancer (NSCLC) and small-cell lung cancer (SCLC), the composition comprising a peptide affinity ligand for a protein of each biomarker of a group of biomarkers, the group of biomarkers comprising CYFRA 21-1, SCC, NSE, Metallopeptidase Inhibitor 1 (TIMP-1) and Her2/Neu.

The invention is based on the finding that the mentioned biomarkers display an altered abundance in certain lung disease scenarios, for example in lung cancer serum samples, when compared to healthy serum samples (biomarkers CYFRA 21-1, Prolactin, SCC, Her2/Neu, optionally also CA125, CEA, CRP, NSE and C4), or for example in lung cancer serum associated with a lung cancer disease showing cancerous lung nodules, when compared with serum samples associated with non-cancerous lung disease showing benign pulmonary nodules (biomarkers CYFRA 21-1, CEA and IL-6), or for example in lung cancer serum associated with lung cancer subtype Non-small-cell lung cancer (NSCLC) when compared with serum samples associated with small-cell lung cancer (SCLC) (biomarkers CYFRA 21-1, SCC, NSE, TIMP-1 and Her2/Neu). These panels provide the advantage that they can be used as standalone panels, e.g. to triage individuals for subsequent imaging-based detection methods, that are not included in the guidelines and to facilitate analysis for individuals that do not follow the regular imaging-based screening schedule etc. They further allow to complement positive and negative findings in a minimally invasive manner.

In a further aspect, the present invention relates to a method of detecting a lung cancer disease in a subject, comprising at least the step of determining the levels of expression of CYFRA 21-1, Prolactin, SCC and Her2/Neu in a sample of the subject. The method may optionally further comprise determining the level of expression of any one or more of CA125, CEA, CRP, NSE and C4 in the sample of the subject.

In a further aspect, the present invention relates to a method of discriminating a non-cancerous lung disease from a lung cancer disease in a subject, comprising at least the step of determining the levels of expression of CYFRA 21-1, CEA and IL-6 in a sample of the subject.

In a further aspect, the present invention relates to a method of discriminating lung cancer subtypes NSCLC and SCLC in a subject, comprising at least the step of determining the levels of expression of CYFRA 21-1, SCC, NSE, TIMP-1 and Her2/Neu in a sample of the subject.

The present invention further relates, in yet another aspect, to a computer-implemented method for the analysis of a sample of a subject comprising the steps: (a) obtaining data on the levels of expression of a group of biomarkers comprising at least CYFRA 21-1, Prolactin, SCC and Her2/Neu and, optionally, also any one or more of CA125, CEA, CRP, NSE and C4, in the subject's sample, preferably from a data storage; (b) inputting the obtained expression data of step (a) into a trained machine learning model wherein the machine learning model is based on a machine learning method such as a logistic regression, linear discriminant analysis (LDA), Random Forest analysis, XGBoost analysis, decision tree analysis or support vector machine (SVM) using the expression data, and wherein the machine learning model is trained based on a training cohort of lung cancer patients and healthy subjects and corresponding diagnostic, prognostic and/or predictive conclusions in the context of lung cancer disease; and (c) deriving a diagnostic, prognostic and/or predictive conclusion output with respect to the detection of the lung cancer disease in the subject.

In a further aspect, the present invention relates to a computer-implemented method for providing a trained machine learning model for the analysis of a sample, comprising the steps: (a) obtaining data on the levels of expression of a group of biomarkers comprising at least CYFRA 21-1, Prolactin, SCC and Her2/Neu and, optionally, also any one or more of CA125, CEA, CRP, NSE and C4, from a cohort of lung cancer patients and healthy subjects from a data storage; (b) performing a machine learning method such as a logistic regression, LDA, Random Forest analysis, XGBoost analysis, decision tree analysis or SVM using the level of expression of CYFRA 21-1, Prolactin, SCC and Her2/Neu and, optionally, also of any one or more of CA125, CEA, CRP, NSE and C4, of the cohort of step (a); and (c) training a machine learning model with the result obtained in step (b) and with diagnostic, prognostic and/or predictive conclusions in the context of lung cancer corresponding to said expression, thereby obtaining a trained machine learning model.

In yet another aspect, the present invention relates to a computer-implemented method for the analysis of a sample of a subject, comprising the steps: (a) obtaining data on the levels of expression of a group of biomarkers comprising CYFRA 21-1, CEA and IL-6 in the subject's sample, preferably from a data storage; (b) inputting the obtained expression data of step (a) into a trained machine learning model, wherein the machine learning model is based on a machine learning method such as a logistic regression, LDA, Random Forest analysis, XGBoost analysis, decision tree analysis or SVM using the expression data, wherein the machine learning model is trained based on a training cohort of patients with a non-cancerous lung disease showing benign pulmonary nodules and of patients with a lung cancer disease showing cancerous lung nodules and with corresponding diagnostic, prognostic and/or predictive conclusions in the context of lung cancer disease or non-cancerous lung disease; and (c) deriving a diagnostic, prognostic and/or predictive conclusion output with respect to the detection of the lung cancer disease or non-cancerous lung disease in the subject.

In yet another aspect, the present invention relates to a computer-implemented method for providing a trained machine learning model for the analysis of a sample, comprising the steps: (a) obtaining data on the levels of expression of a group of biomarkers comprising CYFRA 21-1, CEA and IL-6 from a cohort of patients with a non-cancerous lung disease showing benign pulmonary nodules and of patients with a lung cancer disease showing cancerous lung nodules from a data storage; (b) performing a machine learning method such as a logistic regression, LDA, Random Forest analysis, XGBoost analysis, decision tree analysis or SVM using the levels of expression of CYFRA 21-1, CEA and IL-6 of the cohort of step (a); (c) training a machine learning model with the result obtained in step (b) and diagnostic, prognostic and/or predictive conclusions in the context of lung cancer disease or non-cancerous lung disease corresponding to said expression, thereby obtaining a trained machine learning model.

In a further aspect, the present invention relates to a computer-implemented method for the analysis of a sample of a subject, comprising the steps:(a) obtaining data on the levels of expression of a group of biomarkers comprising CYFRA 21-1, SCC, NSE, TIMP-1 and Her2/Neu in the subject's sample, preferably from a data storage; (b) inputting the obtained expression data of step (a) into a trained machine learning model, wherein the machine learning model is based on a machine learning method such as a logistic regression, LDA, Random Forest analysis, XGBoost analysis, decision tree analysis or SVM using the expression data, wherein the machine learning model is trained based on a training cohort of patients with lung cancer subtypes NSCLC and of patients with SCLC and corresponding diagnostic, prognostic and/or predictive conclusions in the context of NSCLC disease or SCLC disease; and deriving a diagnostic, prognostic and/or predictive conclusion output with respect to the detection of the lung cancer subtypes NSCLC or SCLC in the subject.

In a further aspect, the present invention relates to a computer-implemented method for providing a trained machine learning model for the analysis of a sample, comprising the steps: (a) obtaining data on the levels of expression of a group of biomarkers comprising CYFRA 21-1, SCC, NSE, TIMP-1 and Her2/Neu from a cohort of patients with lung cancer subtype NSCLC and of patients with cancer subtype SCLC from a data storage; (b) performing a machine learning method such as a logistic regression, LDA, Random Forest analysis, XGBoost analysis, decision tree analysis or SVM using the levels of expression CYFRA 21-1, SCC, NSE, TIMP-1 and Her2/Neu of the cohort of step (a); (c) training a machine learning model with the result obtained in step (b) and with diagnostic, prognostic and/or predictive conclusions in the context of lung cancer subtypes NSCLC or SCLC corresponding to said expression, thereby obtaining a trained machine learning model.

In a preferred embodiment of the computer-implemented methods as defined above, the training of the machine learning model additionally includes the inputting of data of the cohorts concerning (i) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (ii) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (iii) detectable molecular changes, preferably on the level of the genome, epigenome, fragmentome, transcriptome, metabolome, proteome, glycome or lipidome and/or laboratory tests.

In a further aspect, the present invention relates to a method of detecting and assessing a lung cancer disease in a subject comprising the steps of: (i) performing the computer-implemented method for the analysis of a sample based on CYFRA 21-1, Prolactin, SCC and Her2/Neu and, optionally, also on any one or more of CA125, CEA, CRP, NSE and C4, as described herein above; (ii) introducing the results obtained in step (i) and optionally introducing (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests into a first machine learning model as obtained in the computer-implemented method based on CYFRA 21-1, Prolactin, SCC and Her2/Neu and, optionally, also on any one or more of CA125, CEA, CRP, NSE and C4 as described herein; (iii) determining with said first machine learning model a likelihood for the presence of a lung cancer disease in the subject; (iv) performing in a subject with a high likelihood as determined in step (iii) the computer-implemented method for the analysis of a sample based on CYFRA 21-1, CEA and IL-6; (v) introducing the results obtained in step (iv) and optionally introducing (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests into a second machine learning model as obtained in the computer-implemented method based on CYFRA 21-1, CEA and IL-6, as described herein; (vi) determining with said second machine learning model a likelihood for the presence of a lung cancer disease vs. a non-cancerous lung disease in the subject; (vii) performing in a subject with a high likelihood of lung cancer disease as determined in step (vi) the computer-implemented method for the analysis of a sample based on CYFRA 21-1, SCC, NSE, TIMP-1 and Her2/Neu, as described herein; (viii) introducing the results obtained in step (vii) and optionally introducing (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests into a third machine learning model as obtained in the computer-implemented method based on CYFRA 21-1, SCC, NSE, TIMP-1 and Her2/Neu, as described herein; and (ix) determining with said third machine learning model the likelihood for the presence of a lung cancer subtype NSCLC or SCLC.

In a particularly preferred embodiment of the present invention, said sample is a body fluid sample, preferably a blood sample such as a serum or plasma sample, an exhaled breath condensate, a breath sample, a saliva sample, or a bronchial brushing sample.

BRIEF DESCRIPTION OF THE DRAWINGS

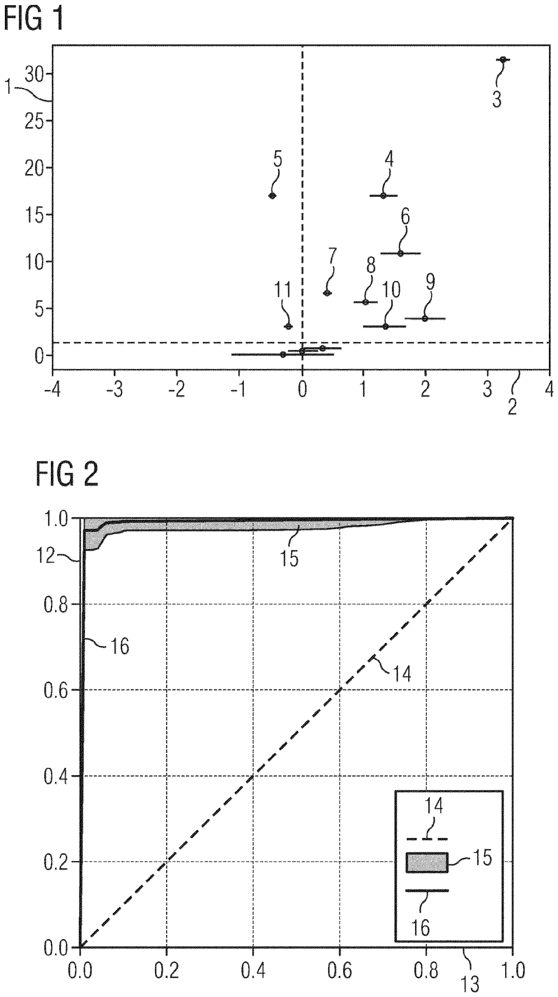

FIG. 1 shows a Volcano plot displaying significantly different abundances of blood-based protein biomarker when comparing patients with lung cancer (n=100, stage I, II, III) to healthy "normal" individuals not known to have cancer (n=100). Significance was calculated with the Mann-Whitney U-test. Indicated are -Log10 p-values (1), Log2 protein abundance ratios (mean (tumor)/mean (normal) (2), Prolactin (PRL) (3), SCC (4), Her2/Neu (5), CYFRA 21-1 (6), NSE (7), CA125 (8), CRP (9), CEA (**10**), C4 (**11**).

FIG. 2 shows performance metrics of the validation set of the multivariate logistic regression model consisting of 9 biomarkers: PRL, SCC, CYFRA21-1, NSE, CA125, CEA, CRP, Her2/Neu, C4. Overall performance of the disease status classification is shown as ROC for a balanced case-control study. On the left-hand side, the scale of the true positive rate (12) is shown. On the abscissa the false positive rate is indicated (13). Further indicated is random (14), as well as +/- 1 std. dev. (15) and the mean ROC (in this Fig. with AUC=0.99 +/- 0.01) (16) .

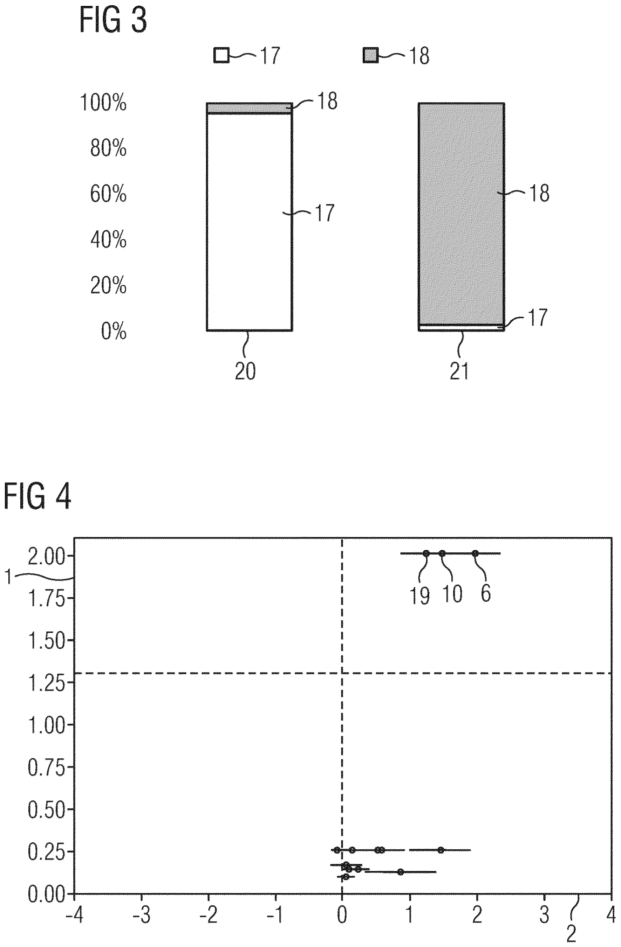

FIG. 3 shows confusion matrix results of correctly classified lung cancer cases across all stages (with lung cancer being indicated as (17)) and healthy cases misclassified as having lung cancer (with normal being indicated as (18)) for the biomarker multi analyte analysis algorithm based on Prolactin, SCC, Her2/Neu, CYFRA 21-1, NSE, CA125, CEA, CRP, CEA and C4. Further indicated are lung cancer cases (20) and normal cases (21) .

FIG. 4 shows a Volcano plot displaying significantly differently abundant blood-based protein biomarkers when comparing patients with lung cancer (n=100, stage I, II, III) to patients with benign, non-cancerous findings in the lung like pneumonia (n=10) or hamartoma (n=10). Significance was calculated with the Mann-Whitney U-test. Indicated are -Log10 p-values (**1**), Log2 protein abundance ratios (mean (tumor)/mean (benign) (**2**), CYFRA 21-1 (**6**), CEA (**10**), and IL-6 (**19**)).

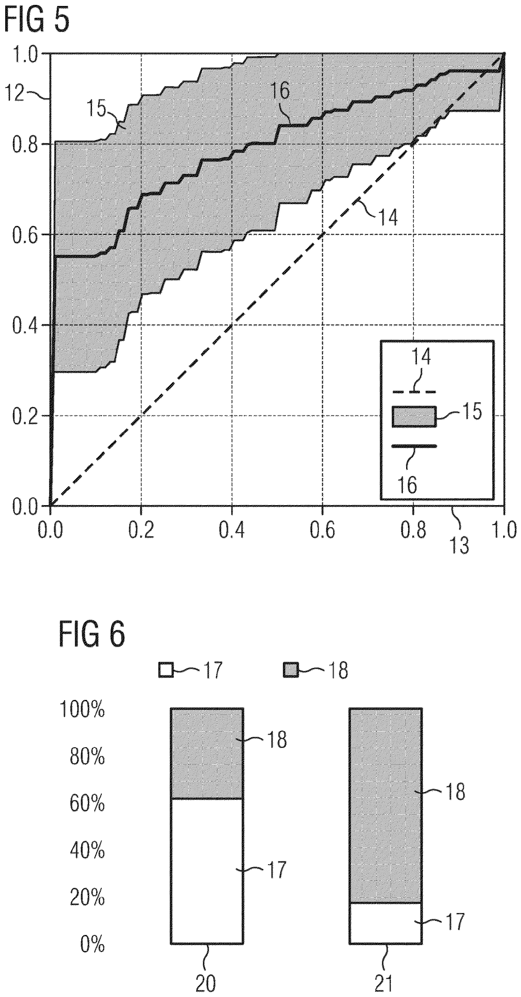

FIG. 5 shows performance metrics of the validation set of the multivariate logistic regression model consisting of 3 biomarkers: CYFRA21-1, CEA, IL-6. Overall performance is shown as ROC for a balanced case-control study. On the left-hand side, the scale of the true positive rate (**12**) is shown. On the abscissa the false positive rate is indicated (**13**). Further indicated is random (**14**), as well as +/- 1 std. dev. (**15**) and the mean ROC (in this Fig. with AUC=0.79 +/- 0.13) (**16**).

FIG. 6 shows confusion matrix results of correctly classified lung cancer cases across all stages (with lung cancer being indicated as (**17**)) and healthy cases misclassified as having lung cancer (with normal being indicated as (**18**)) for the biomarker multi analyte analysis algorithm based on: CYFRA21-1, CEA and IL-6. Further indicated are lung cancer cases (**20**) and normal cases (**21**).

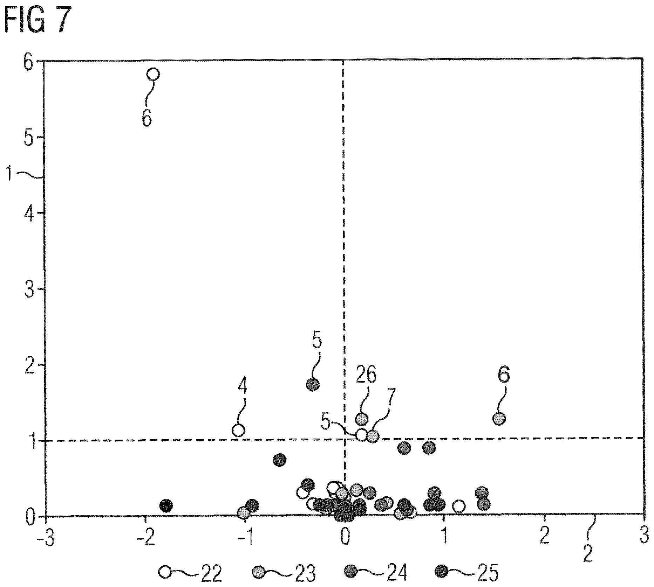

FIG. 7 shows a Volcano plot showing differently abundant blood-based protein biomarkers when comparing patients with a specific lung cancer subtype to all other lung cancer subtypes. Significance was calculated with the Mann-Whitney U-test. An exploratory threshold of p<0.1 was selected for biomarker discovery. Indicated are -Log10 p-values (**1**), Log2 protein abundance ratios (mean (specific cancer subtype)/mean (all other cancer subtypes) (**2**), SCC (**4**), Her2/Neu (**5**), CYFRA 21-1 (**6**), NSE (**7**), TIMP-1 (**26**). The shown data points correspond to NSCLC squamous cell carcinoma vs. all other subtypes (**22**), NSCLC adenocarcinoma vs. all other subtypes (**23**), SCLC vs. all other subtypes (**24**) and SCLC neuroendocrine subset vs. all other subtypes (**25**).

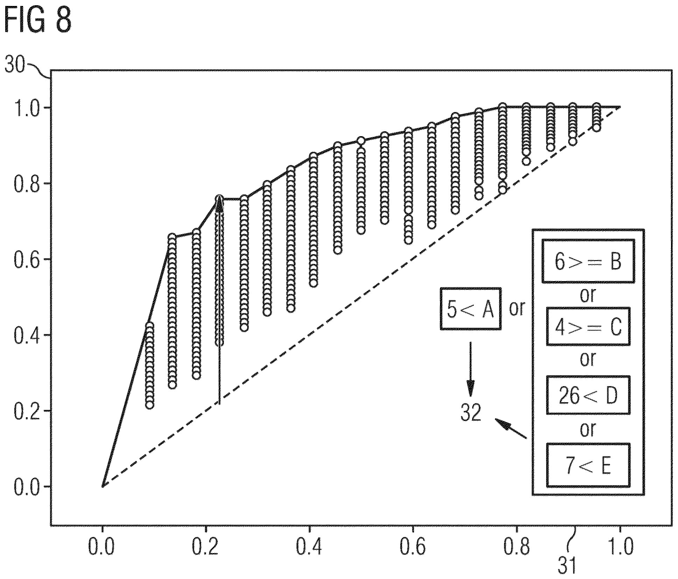

FIG. 8 depicts the determined sensitivities/false positive rates for Brute-Force variation of all thresholds of the protein biomarkers Her2/Neu (**5**), CYFRA21-1 (**6**), SCC (**4**), TIMP-1 (**26**), and NSE (**7**) in a diagnostic algorithm following the logic depicted below the diagonal "random guess line". The threshold combination which has the largest distance from the diagonal is deemed optimal (black arrow). Indicated are sensitivity (**30**) and false positive rate (**31**), as well as NSCLC (**32**) as output of the diagnostic algorithm.

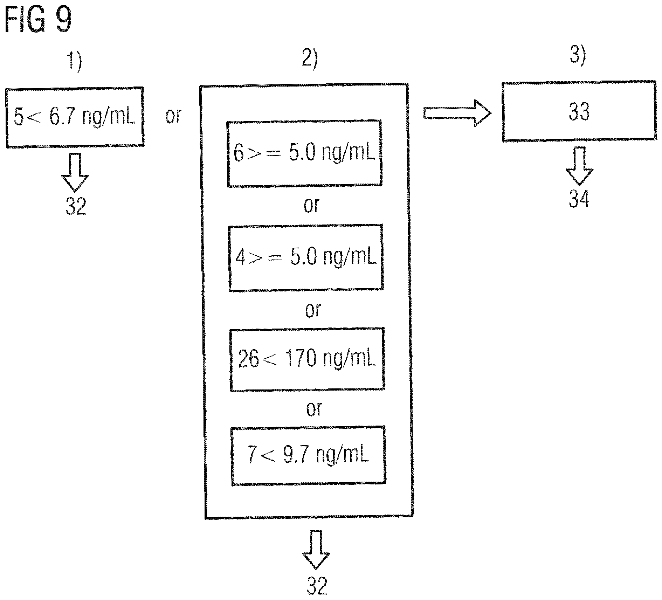

FIG. 9 shows a preferred diagnostic algorithm with the optimal biomarker thresholds. It has a sensitivity of 0.76 and a specificity of 0.77 (1- False Positive Rate) on the obtained data. Indicated are the protein biomarkers Her2/Neu (**5**), CYFRA21-1 (**6**), SCC (**4**), TIMP-1 (**26**), and NSE (**7**), as well as the output of the diagnostic algorithm: likely NSCLC (**32**) and the requirement "if condition 1 or 2 are not indicative of NSCLC" (**33**) and likely SCLC (**34**).

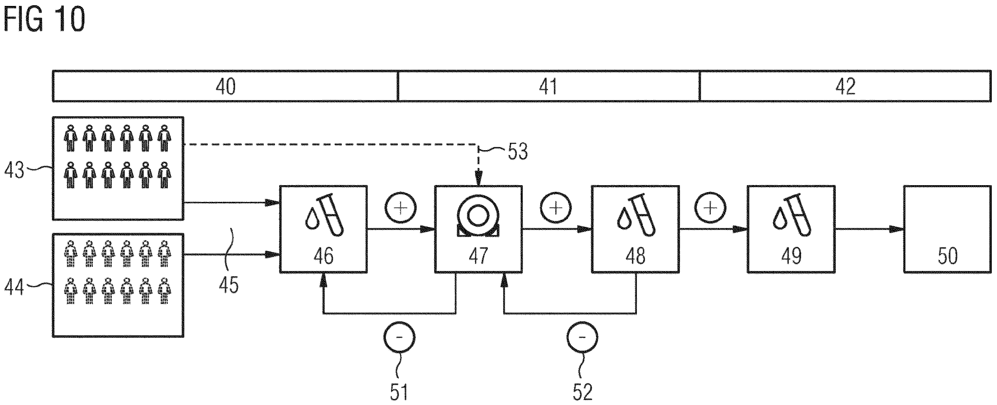

FIG. 10 shows an overall scheme for the detection of lung cancer according to embodiments of the present invention. Indicated are the phases seek (**40**), assess (**41**) and subtype (**42**). Further indicated are high risk (**43**), general risk (**44**) and personal criteria (**45**), leading to a first machine learning model (**46**), a possible CT scan (**47**), a second machine learning model (**48**), a third machine learning model (**49**) and a diagnostic work-up (**50**). In addition, the steps of a periodical follow-up with laboratory tests (**51**) and a follow-up by diagnostic imaging (**52**) are indicated.

## DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of $\pm 20$ %, preferably $\pm 15$ %, more preferably $\pm 10$ %, and even more preferably $\pm 5$ %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", "(iii)" or " (a) ", "(b)", "(c)", "(d)", or "first", "second", "third" etc. and the like in the description or in the claims, are used for distinguishing between similar or structural elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e., the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a composition for detecting a lung cancer disease, comprising peptide affinity ligands for the proteins of a group of biomarkers comprising at least CYFRA 21-1, Prolactin, SCC and Her2/Neu.

The term "peptide affinity ligand for the CYFRA 21-1 protein" as used herein refers to a peptide molecule being able to specifically bind to the CYFRA 21-1 protein. The peptide molecule may preferably be able to specifically bind to a protein or polypeptide comprising the amino acid sequence as set forth in UniProt Reference number P08727. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_002276.5 or to a protein or polypeptide comprising an amino acid sequence being at least 90%, 91%, 92%, 93%, 94%, 95%, 96%, 97%, 98% or 99% identical to the sequence as set forth in UniProt Reference number P08727 or to any fragments of said sequences. Further envisaged are splice or modification variants to the above referenced sequences as known to the skilled person or derivable from suitable databases.

The term "peptide affinity ligand for the Prolactin protein" as used herein refers to a peptide molecule being able to specifically bind to the Prolactin protein. The peptide molecule may preferably be able to specifically bind to a protein or polypeptide comprising the amino acid sequence as set forth in UniProt Reference number P01236. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000948.6 or to a protein or polypeptide comprising an amino acid sequence being at least 90%, 91%, 92%, 93%, 94%, 95%, 96%, 97%, 98% or 99% identical to the sequence as set forth in UniProt Reference number P01236 or to any fragments of said sequences. Further envisaged are splice or modification variants to the above referenced sequences as known to the skilled person or derivable from suitable databases.

The term "peptide affinity ligand for the Squamous Cell Carcinoma Antigen (SCC) protein" as used herein refers to a peptide molecule being able to specifically bind to the SCC protein. The peptide molecule may preferably be able to specifically bind to a protein or polypeptide comprising the amino acid sequence as set forth in UniProt Reference number Q15020. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_014706.4 or to a protein or polypeptide comprising an amino acid sequence being at least 90%, 91%, 92%, 93%, 94%, 95%, 96%, 97%, 98% or 99% identical to the sequence as set forth in UniProt Reference number Q15020 or to any fragments of said sequences. Further envisaged are splice or modification variants to the above referenced sequences as known to the skilled person or derivable from suitable databases.

The term "peptide affinity ligand for the Her2/Neu protein" as used herein refers to a peptide molecule being able to specifically bind to the Her2/Neu protein. The peptide molecule may preferably be able to specifically bind to a protein or polypeptide comprising the amino acid sequence as set forth in UniProt Reference number P04626. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_001005862.3 or to a protein or polypeptide comprising an amino acid sequence being at least 90%, 91%, 92%, 93%, 94%, 95%, 96%, 97%, 98% or 99% identical to the sequence as set forth in UniProt Reference number P04626 or to any fragments of said sequences. Further envisaged are splice or modification variants to the above referenced sequences as known to the skilled person or derivable from suitable databases.

The term "peptide" refers to any type of amino acid sequence comprising more than 2 amino acids, e.g. polypeptide structures, protein structures, protein or functional derivatives thereof. Furthermore, the peptide may, in certain embodiments, be combined with further chemical moieties or functionalities.

The term "lung cancer disease" as used herein refers to a lung cancer or uncontrolled malignant transformation and proliferation of lung tissue including all kind of subtypes. Such subtypes can be histological subtypes like Non-Small Cell Lung Cancer (NSCLC), Small Cell Lung Cancer (SCLC) or molecularly defined subtypes. Further, the lung cancer disease may further include the presence of pre-invasive lesions or a pre-cancerous cell population, or a predisposition for cancer or tumor. In certain embodiments, the lung cancer is an early form of lung cancer, e.g. a lung cancer of stage I or II.

The term "detecting a lung cancer disease" as used herein means that the presence of a lung cancer disease or disorder in an organism may be determined or identified. The determination or identification of a lung cancer disease or disorder may, in certain embodiments, be accomplished by a comparison of the expression levels, e.g. protein concentrations, of the biomarkers of the present invention with control levels. A lung cancer may be detected when the level of expression of the group of biomarkers according to the present invention comprising at least CYFRA 21-1, Prolactin, SCC and Her2/Neu is changed in comparison to a control level. In a specific embodiment of the present invention, a lung cancer may be detected if the expression level of the group of biomarkers according to the present invention comprising at least CYFRA 21-1, Prolactin, SCC and Her2/Neu is similar to an expression level of a suitable cancer reference sample.

In further embodiments, the composition for detecting a lung cancer disease according to the present invention additionally comprises a peptide affinity ligand for a protein of one or more of CA125, CEA, CRP, NSE and C4. For example, the composition may comprise peptide affinity ligands for the proteins CYFRA 21-1, Prolactin, SCC and Her2/Neu and any 1, 2, 3, 4 or 5 of CA125, CEA, CRP, NSE and C4.

For example, in one embodiment, the composition may comprise peptide affinity ligands for the proteins of CYFRA 21-1, Prolactin, SCC and Her2/Neu and CA125.

In a further embodiment, the composition may comprise peptide affinity ligands for the proteins of CYFRA 21-1, Prolactin, SCC and Her2/Neu and CEA.

In a further embodiment, the composition may comprise peptide affinity ligands for the proteins of CYFRA 21-1, Prolactin, SCC and Her2/Neu and CRP.

In a further embodiment, the composition may comprise peptide affinity ligands for the proteins of CYFRA 21-1, Prolactin, SCC and Her2/Neu and NSE.

In a further embodiment, the composition may comprise peptide affinity ligands for the proteins of CYFRA 21-1, Prolactin, SCC and Her2/Neu and C4.

In yet another group of embodiments, the composition may comprise peptide affinity ligands for the proteins of CYFRA 21-1, Prolactin, SCC and Her2/Neu and CA125 and CEA, or of CYFRA 21-1, Prolactin, SCC and Her2/Neu and CEA and CRP, or of CYFRA 21-1, Prolactin, SCC and Her2/Neu and CRP and NSE, or of CYFRA 21-1, Prolactin, SCC and Her2/Neu and NSE and C4, or of CYFRA 21-1, Prolactin, SCC and Her2/Neu and CA125 and CRP, or of CYFRA 21-1, Prolactin, SCC and Her2/Neu and CA125 and NSE, or of CYFRA 21-1, Prolactin, SCC and Her2/Neu and CA125 and C4, or of CYFRA 21-1, Prolactin, SCC and Her2/Neu and CEA and NSE, or of CYFRA 21-1, Prolactin, SCC and Her2/Neu and CEA and C4, or of CYFRA 21-1, Prolactin, SCC and Her2/Neu and CRP and C4.

In yet another group of embodiments, the composition may comprise peptide affinity ligands for the proteins of CYFRA 21-1, Prolactin, SCC and Her2/Neu, and CA125 and CEA and CRP, or of CYFRA 21-1, Prolactin, SCC and Her2/Neu, and CA125 and CEA and NES, or of CYFRA 21-1, Prolactin, SCC and Her2/Neu, and CA125 and CEA and C4, or of CYFRA 21-1, Prolactin, SCC and Her2/Neu, and CEA and CRP and NSE, or of CYFRA 21-1, Prolactin, SCC and Her2/Neu, and CEA and CRP and C4, or of CYFRA 21-1, Prolactin, SCC and Her2/Neu, and CRP and NSE and C4.

In yet another group of embodiments, the composition may comprise peptide affinity ligands for the proteins of CYFRA 21-1, Prolactin, SCC and Her2/Neu, and CA125 and CEA and CRP and NSE, or of CYFRA 21-1, Prolactin, SCC and Her2/Neu, and CA125 and CEA and CRP and C4, or of CYFRA 21-1, Prolactin, SCC and Her2/Neu, and CEA and CRP and NSE and C4, or of CYFRA 21-1, Prolactin, SCC and Her2/Neu, and CRP and NSE and CA125 and C4, or of CYFRA 21-1, Prolactin, SCC and Her2/Neu, and NSE and C4 and CA125 and CEA.

In yet another group of embodiments, the composition may comprise peptide affinity ligands for the proteins of CYFRA 21-1, Prolactin, SCC and Her2/Neu, and CA125 and CEA and CRP and NSE and C4.

The term "peptide affinity ligand for the cancer antigen 125 (CA125) protein" as used herein refers to a peptide molecule being able to specifically bind to the CA125 protein. The peptide molecule may preferably be able to specifically bind to a protein or polypeptide comprising the amino acid sequence as set forth in UniProt Reference number Q8WXI7. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_024690.2 or to a protein or polypeptide comprising an amino acid sequence being at least 90%, 91%, 92%, 93%, 94%, 95%, 96%, 97%, 98% or 99% identical to the sequence as set forth in UniProt Reference number Q8WXI7 or to any fragments of said sequences. Further envisaged are splice or modification variants to the above referenced sequences as known to the skilled person or derivable from suitable databases.

The term "peptide affinity ligand for the carcinoembryonic antigen (CEA) protein" as used herein refers to a peptide molecule being able to specifically bind to the CEA protein. The peptide molecule may preferably be able to specifically bind to a protein or polypeptide comprising the amino acid sequence as set forth in UniProt Reference number P06731. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_004363.6 or to a protein or polypeptide comprising an amino acid sequence being at least 90%, 91%, 92%, 93%, 94%, 95%, 96%, 97%, 98% or 99% identical to the sequence as set forth in UniProt Reference number P06731 or to any fragments of said sequences. Further envisaged are splice or modification variants to the above referenced sequences as known to the skilled person or derivable from suitable databases.

The term "peptide affinity ligand for the C-reactive protein (CRP) protein" as used herein refers to a peptide molecule being able to specifically bind to the CRP protein. The peptide molecule may preferably be able to specifically bind to a protein or polypeptide comprising the amino acid sequence as set forth in UniProt Reference number P02741. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000567.3 or to a protein or polypeptide comprising an amino acid sequence being at least 90%, 91%, 92%, 93%, 94%, 95%, 96%, 97%, 98% or 99% identical to the sequence as set forth in UniProt Reference number P02741 or to any fragments of said sequences. Further envisaged are splice or modification variants to the above referenced sequences as known to the skilled person or derivable from suitable databases.

The term "peptide affinity ligand for the Neuron Specific Enolase (NSE) protein" as used herein refers to a peptide molecule being able to specifically bind to the NSE protein. The peptide molecule may preferably be able to specifically bind to a protein or polypeptide comprising the amino acid sequence as set forth in UniProt Reference number P09104. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_001975.3 or to a protein or polypeptide comprising an amino acid sequence being at least 90%, 91%, 92%, 93%, 94%, 95%, 96%, 97%, 98% or 99% identical to the sequence as set forth in UniProt Reference number P09104 or to any fragments of said sequences. Further envisaged are splice or modification variants to the above referenced sequences as known to the skilled person or derivable from suitable databases.

The term "peptide affinity ligand for the C4 complement (C4) protein" as used herein refers to a peptide molecule being able to specifically bind to the C4 complement protein. The peptide molecule may preferably be able to specifically bind to a protein or polypeptide comprising the amino acid sequence as set forth in UniProt Reference number P0C0L4 or P0C0L5. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_007293.3 or NM_001002029.4, respectively, or to a protein or polypeptide comprising an amino acid sequence being at least 90%, 91%, 92%, 93%, 94%, 95%, 96%, 97%, 98% or 99% identical to the sequence as set forth in UniProt Reference number P0C0L4 or P0C0L5, respectively, or to any fragments of said sequences. Further envisaged are splice or modification variants to the above referenced sequences as known to the skilled person or derivable from suitable databases.

In preferred embodiments, the peptide affinity ligand is an antibody specific for CYFRA 21-1, Prolactin, SCC, Her2/Neu, CA125, CEA, CRP, NSE or C4.

In a further aspect, the invention relates to a composition for discriminating a non-cancerous lung disease showing benign pulmonary nodules from a lung cancer disease showing cancerous lung nodules, comprising a peptide affinity ligand for a protein of each biomarker of a group of biomarkers, the group of biomarkers comprising CYFRA 21-1, CEA and Interleukin 6 (IL-6).

The terms "peptide affinity ligand for the CYFRA 21-1 protein" and "peptide affinity ligand for the CEA protein" have been defined herein above.

The term "peptide affinity ligand for the Interleukin 6 (IL-6) protein" as used herein refers to a peptide molecule being able to specifically bind to the IL-6 protein. The peptide molecule may preferably be able to specifically bind to a protein or polypeptide comprising the amino acid sequence as set forth in UniProt Reference number P05231. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_000600.5 or to a protein or polypeptide comprising an amino acid sequence being at least 90%, 91%, 92%, 93%, 94%, 95%, 96%, 97%, 98% or 99% identical to the sequence as set forth in UniProt Reference number P05231 or to any fragments of said sequences. Further envisaged are splice or modification variants to the above referenced sequences as known to the skilled person or derivable from suitable databases.

The term "lung cancer disease showing cancerous lung nodules" as used herein refers to a lung cancer or uncontrolled malignant transformation and proliferation of lung tissue, which is in a specific cancerous state and displays, typically upon imaging analysis such as X-ray or CT as pulmonary or lung nodules. A pulmonary nodule is typically a small, roundish growth on the lung having a diameter of 3 cm or less, which might be benign or cancerous, wherein up to 40 % of detected nodules turn out to be cancerous. Based on the specific biomarker group according to the present invention, the disease state of lung cancer disease showing cancerous lung nodules can be discriminated from a non-cancerous lung diseases showing benign pulmonary entities or nodules. A non-cancerous disease showing or leading to pulmonary nodules may, for example, be a hamartoma or a fungal or bacterial infection, such as tuberculosis, leading to a granuloma, or an autoimmune disease, such as rheumatoid arthritis and sarcoidosis.

The term "discriminating a non-cancerous lung disease showing benign pulmonary nodules from a lung cancer disease showing cancerous lung nodules" as used herein means that the presence of a lung cancer disease showing cancerous lung nodules in an organism may be determined or that such a disease or disorder may be differentiated from a similarly looking benign nodule, i.e. from a non-cancerous lung disease showing benign pulmonary nodules in an organism. The discrimination or determination of a lung cancer disease showing cancerous lung nodules may, in certain embodiments, be accomplished by a comparison of the expression levels, e.g. protein concentrations, of the biomarkers of the present invention with control levels. A lung cancer disease showing cancerous lung nodules may be detected when the level of expression of the group of biomarkers according to the present invention comprising CYFRA 21-1, CEA and IL-6 is changed in comparison to a control level, e.g. a level as present in a non-cancerous lung disease showing benign pulmonary nodules. In a specific embodiment of the present invention, a lung cancer disease showing cancerous lung nodules may be detected if the expression level of the group of biomarkers according to the present invention comprising CYFRA 21-1, CEA and IL-6 is similar to an expression level of a suitable cancer reference sample, preferably an expression level of a reference cell derived from a subject affected by lung cancer disease showing cancerous lung nodules.

In preferred embodiments, the peptide affinity ligand is an antibody specific for CYFRA 21-1, CEA or IL-6.

In a further aspect, the invention relates to a composition for discriminating lung cancer subtypes Non-small-cell lung cancer (NSCLC) and small-cell lung cancer (SCLC), comprising a peptide affinity ligand for a protein of each biomarker of a group of biomarkers, the group of biomarkers comprising at least CYFRA 21-1, SCC, NSE, Metallopeptidase Inhibitor 1 (TIMP-1) and Her2/Neu.

The terms "peptide affinity ligand for the CYFRA 21-1 protein", "peptide affinity ligand for the SCC protein", "peptide affinity ligand for the NSE protein" and "peptide affinity ligand for the Her2/Neu protein" have been defined herein above.

The term "peptide affinity ligand for the Metallopeptidase Inhibitor 1 (TIMP-1) protein" as used herein refers to a peptide molecule being able to specifically bind to the TIMP-1 protein. The peptide molecule may preferably be able to specifically bind to a protein or polypeptide comprising the amino acid sequence as set forth in UniProt Reference number P01033. The peptide affinity ligand may also be able to specifically bind to a protein or polypeptide comprising an amino acid sequence encoded by a DNA sequence being at least 90%, 91%, 92%, 93%, 94%, 95%, 96%, 97%, 98% or 99% identical to the sequence as set forth in NCBI Reference number NM_003254.3 or to a protein or polypeptide comprising an amino acid sequence being at least 90%, 91%, 92%, 93%, 94%, 95%, 96%, 97%, 98% or 99% identical to the sequence as set forth in UniProt Reference number P01033 or to any fragments of said sequences. Further envisaged are splice or modification variants to the above referenced sequences as known to the skilled person or derivable from suitable databases.

The term "small-cell lung cancer (SCLC)" refers to a highly malignant cancer that commonly arises within the lung and represents 15% of all lung cancer cases. SCLC is an undifferentiated neoplasm composed of primitive-appearing cells. Typically, the cells in SCLC are smaller than normal cells and show limited cytoplasm. SCLC usually presents in the central airways and infiltrates the submucosa leading to narrowing of bronchial airways. Common symptoms include cough, dyspnea, weight loss, and debility. The term "Non-small-cell lung cancer (NSCLC)" as used herein refers to any type of epithelial lung cancer other than small-cell lung cancer (SCLC). Compared to NSCLC, SCLC is more aggressive, with a shorter doubling time, higher growth fraction, and earlier development of metastases. The most common types of NSCLC are squamous-cell carcinoma, large-cell carcinoma, and adenocarcinoma, representing 25%, 10% and 40% of all lung cancer cases. Based on the specific biomarker group according to the present invention, the disease state of NSCLC can be discriminated from SCLC.

The term "discriminating NSCLC from SCLC" as used herein means that the presence of an NSCLC lung cancer disease in an organism may be determined or that such a disease or disorder may be differentiated from an SCLC in an organism. The discrimination or determination of a NSCLC or SCLC may, in certain embodiments, be accomplished by a comparison of the expression levels, e.g. protein concentrations, of the biomarkers of the present invention with control levels. An NSCLC may be detected when the level of expression of the group of biomarkers comprising CYFRA 21-1, SCC, NSE, Metallopeptidase Inhibitor 1 (TIMP-1) and Her2/Neu is changed in comparison to a control level, e.g., a level as present in SCLC. In a specific embodiment of the present invention an NSCLC may be detected if the expression level of the group of biomarkers comprising CYFRA 21-1, SCC, NSE, Metallopeptidase Inhibitor 1 (TIMP-1) and Her2/Neu is similar to an expression level of a suitable cancer reference sample, preferably an expression level of a reference cell derived from a subject affected by NSCLC. Similarly, in a further specific embodiment of the present invention, an SCLC may be detected if the expression level of the group of biomarkers comprising CYFRA 21-1, SCC, NSE, Metallopeptidase Inhibitor 1 (TIMP-1) and Her2/Neu is similar to an expression level of a suitable cancer reference sample, preferably an expression level of a reference cell derived from a subject affected by SCLC.

In preferred embodiments, the peptide affinity ligand is an antibody specific for CYFRA 21-1, SCC, NSE, Metallopeptidase Inhibitor 1 (TIMP-1) or Her2/Neu.

In certain embodiments, a detected or identified lung cancer disease or cancerous lung nodule, NSCLC or SCLC, may be monitored. The term "monitoring of a lung cancer disease, cancerous lung nodule, NSCLC or SCLC" as used herein relates to the accompaniment of detected lung cancer diseases. The accompaniment may be performed during a treatment procedure or during a certain period of time, typically during 2 months, 3 months, 4 months, 6 months, 1 year, 2 years, 3 years, 5 years, 10 years, or any other period of time. The term "accompaniment" means that states of disease, e.g. stage I or II lung cancer, in particular, changes of these states of disease may be detected by comparing the levels of expression, e.g. concentrations of proteins, of the group of biomarkers comprising (i) at least CYFRA 21-1, Prolactin, SCC and Her2/Neu and, optionally, also any one or more of CA125, CEA, CRP, NSE and C4; or (ii) CYFRA 21-1, CEA and IL-6; or (iii) CYFRA 21-1, SCC, NSE, TIMP-1 and Her2/Neu in a sample of a subject to a control level such as, for example, a previously or initially measured level of a patient, e.g. within the time frame(s) mentioned above. The control level can, in certain embodiments, be a baseline level that was measured in the respective subject at the beginning of a series of measurements and with which all subsequent measurements are compared. Alternatively, a control level of expression can be derived from a healthy control or the level of expression of an established, e.g. independently established, cancer cell or cell line, e.g. a lung cancer cell line according to the above mentioned disease definitions, in any type of periodical time segment, e.g. every week, every 2 weeks, every month, every 2, 3, 4, 5, 6, 7, 8, 9, 10, 11 or 12 month, every 1.5 year, every 2, 3, 4, 5, 6, 7, 8,9 or 10 years, during any period of time, e.g. during 2 weeks, 3 weeks, 1, 2, 3, 4, 5, 6, 7, 8, 9, 10, 11, 12 months, 1.5, 2, 3, 4, 5, 6, 7, 8, 9, 10, 15 or 20 years, respectively. The established, e.g. independently established, cancer cell, e.g. lung cancer cell, SCLC, NSCLC, or cell line giving rise to an additional control level may be derived from samples corresponding to different stages of cancer development, e.g. stages I and II of the AJCC staging system. In a specific embodiment of the present invention, the term relates to the accompaniment of a diagnosed lung cancer disease.

In a further aspect, the present invention relates to a method of detecting a lung cancer disease in a subject, comprising at least the step of determining the levels of expression of CYFRA 21-1, Prolactin, SCC and Her2/Neu and, optionally, also of any one or more of CA125, CEA, CRP, NSE and C4 in a sample of the subject.

Also envisaged is a method of discriminating a non-cancerous lung disease from a lung cancer disease in a subject, comprising at least the step of determining the levels of expression of CYFRA 21-1, CEA and IL-6 in a sample of the subject, as well as a method of discriminating lung cancer subtypes NSCLC and SCLC in a subject, comprising at least the step of determining the levels of expression of CYFRA 21-1, SCC, NSE, TIMP-1 and Her2/Neu in a sample of the subject.

The term "level of expression" as used herein refers to the amount of protein or polypeptide, preferably measured as concentration of protein or polypeptide, of the biomarkers CYFRA 21-1, Prolactin, SCC, Her2/Neu, CA125, CEA, CRP, NSE, C4, IL-6 or TIMP-1 as defined herein above.

The present invention is thus based on the determination of the amount, i.e. concentration, of proteins corresponding to the different biomarkers disclosed herein.

In certain embodiments, the amount of nucleic acids, e.g. transcript or RNA or cDNA derived therefrom may be determined. Also envisaged is the detection of miRNAs associated with the herein described markers. In further special embodiments, both, the amount of mRNAs or miRNAs and proteins may be determined.

The term "sample" as used herein refers to any suitable sample type or form. In preferred embodiments, the sample is a biological sample. It is further preferred that the sample is a body fluid sample, such as a blood sample, including a serum or plasma sample. In further embodiments, the sample may be an exhaled breath condensate, a breath sample, a saliva sample, or a bronchial brushing sample.

In certain embodiments, the sample may be a tumor or lung nodule sample, i.e., the proteins may be extracted from a tumor of a subject, e.g. a lung tumor, e.g. NSCLC or SCLC, or from a cancerous lung nodule. In other embodiments, the sample may be derived from a subject having a lung disease showing benign pulmonary nodules or from a healthy subject.

The term "subject" as used herein refers to an individual such as a healthy individual that is not suspected of having a pre-invasive lesion, a pre-cancerous cell or a cancer disease; or an individual that is not suspected of having a pre-invasive lesion, a pre-cancerous cell or a cancer disease, e.g. lung cancer; or an individual that is suspected of having a pre-invasive lesion or a pre-cancerous cell; or an individual, that has a pre-invasive lesion or a pre-cancerous cell population but is not suspected of having a cancer disease, e.g. lung cancer; or an individual that has a pre-invasive lesion or a pre-cancerous cell population and is suspected of having a cancer disease, e.g. lung cancer; or an individual that is suspected of having a pre-invasive lesion or a pre-cancerous cell population; or an individual that has a pre-invasive lesion or a pre-cancerous cell population; or an individual that is suspected of having a lung cancer disease; or an individual that has a lung cancer disease. The subject may, in certain embodiments, also be an individual which has been diagnosed to have a lung cancer disease but not yet for the presence of NSCLC or SCLC; or an individual which may show or be suspected of having cancerous pulmonary nodules; or an individual which may show pulmonary nodules but is not suspected of having a lung cancer disease; or an individual which may show pulmonary nodules and is suspected of having a lung cancer disease.

The term "patient" as used herein is meant to relate to an individual that has a pre-invasive lesion or a pre-cancerous cell population; or an individual that is afflicted by a lunger cancer disease, e.g. NSCLC or SCLC.

To "determine the level of expression" the amount of expression products, i.e. of protein, is measured. Thus, the expression level may be determined by methods involving the detection of a protein, preferably the concentration of the protein.

The measurement of protein levels or concentrations of the biomarkers of the present invention or of any fragments, homologues or derivates derived thereof may be carried out via any suitable detection technique known in the art. Preferably, the protein level or concentration of the biomarkers of the present invention may be determined immunologically, e.g. by using an antibody specific for the respective biomarker. Alternatively, antibody variants or fragments may be used or oligomeric binders like aptamers.

Preferably, a peptide affinity ligand according to the present invention as defined herein above may be used.

Within the context of the present invention, specific antibodies may be used either in an immunoturbidimetry assay or in a "sandwich" immunoassay or in a combination of both assays in an integrated analyzer. For the immunoturbidimetry assay the antibody may be immobilized on a solid support. Such solid support could be a particle, preferably a bead. Samples to be analyzed may be mixed with antibodies resulting in an agglutination of the antibodies and a turbid solution. The turbidity of the solution can be quantified by a spectroscopic technique, preferably UV/Vis spectrometry. For the "sandwich" immunoassay a first antibody may be immobilized on a solid support, e.g. on a particle, preferably a bead. A second antibody may be labelled with a reporter. Samples to be analyzed may subsequently be mixed with the two types of antibodies. The detection process may then be carried out with the reporter to quantify the protein expression levels. The reporter could be a radiolabel, an enzyme, a fluorescent molecule, a chemiluminescent molecule, such as acridinium esters, an electrochemiluminescent molecule, a mass tag or a nucleic acid tag. Further details would be known to the skilled person or can be derived from suitable literature sources. In some embodiments, the reporter amount can be determined by quantifying the nucleic acid tag by next generation sequencing, quantitative PCR or digital PCR.

Immunological tests which may be used in the context of the present invention, in particular for the diagnostic purposes of the present invention, include, for example, competitive and non-competitive assay systems using techniques such as Western blots, radioimmunoassay like RIA (radio-linked immunoassay), ELISA (enzyme linked immunosorbent assay), "sandwich" immunoassays, immunoprecipitation assays, precipitation reactions, gel diffusion precipitation reactions, immunodiffusion assays, agglutination assays, e.g. latex agglutination, complement-fixation assays, immunoradiometric assays, fluorescent immunoassays, e.g. FIA (fluorescence-linked immunoassay), chemiluminescence immunoassays, e.g. acridinium ester chemiluminometric immunoassays, electrochemiluminescence immunoassays (ECLIA) and protein A immunoassays. Such assays are routine and well known to the person skilled in the art.

Furthermore, the binding affinity of an antibody to an antigen and the off-rate of an antibody-antigen interaction may be determined by competitive binding assays. One example of a competitive binding assay is a radioimmunoassay comprising the incubation of labeled antigen (e.g., $^3$H or $^{125}$I) with a suitable antibody in the presence of increasing amounts of unlabeled antigen, and the detection of the antibody bound to the labeled antigen. The affinity of the antibody of interest for a particular antigen and the binding off-rates may be determined from the data by any suitable analysis approach, e.g. by a scatchard plot analysis. Competition with a second antibody may also be determined using radioimmunoassays. In this case, the antigen may be incubated with a suitable antibody conjugated to a labeled compound (e.g., $^3$H or $^{125}$I) in the presence of increasing amounts of an unlabeled second antibody.

In certain embodiments, the methods as defined above may comprise an additional step of testing in a control sample the levels of expression of (i) CYFRA 21-1, Prolactin, SCC and Her2/Neu and, optionally, also of any one or more of CA125, CEA, CRP, NSE and C4; or (ii) CYFRA 21-1, CEA and IL-6; or (iii) CYFRA 21-1, SCC, NSE, TIMP-1 and Her2/Neu, and determining the difference of the levels of expression of (i) CYFRA 21-1, Prolactin, SCC and Her2/Neu and, optionally also of any one or more of CA125, CEA, CRP, NSE and C4; or (ii) CYFRA 21-1, CEA and IL-6; or (iii) CYFRA 21-1, SCC, NSE, TIMP-1 and Her2/Neu in a subject's sample vs. the control sample.

The term "control sample" relates to a sample from the same subject as the test sample, or to a sample derived from a different source or subject. The control sample may further be either a sample derived from the same tissue or be derived from a different tissue type. Furthermore, the testing of the test sample for the expression of a reference gene or protein and the testing of a control sample may be combined. It is preferred that the control sample is the sample of a healthy subject, i.e. a subject which is not affected by a disorder or disease or condition as mentioned herein, e.g. lung cancer disease. In alternative embodiments, the control sample may be a sample derived from a cancerous subject, e.g. from a subject affected by a disorder or disease or condition as mentioned herein, e.g. lung cancer, a lung cancer disease showing cancerous lung nodules, NSCLC or SCLC, or from a collection of cancerous samples.

The use of control samples may, in certain embodiments, yield control levels of expression of the tested biomarkers. Accordingly, the term "control level" as used herein, relates to an expression level which may be determined at the same time and/or under similar or comparable conditions as the test sample by using, for example, one or more samples previously collected and stored from a subject/subjects whose condition or disease state, e.g. non-cancerous, normal or healthy is/are known, e.g. have been determined by an independent molecular, histological or physiological method known to the person skilled in the art.

By comparing the results obtained by determining the levels of expression of (i) CYFRA 21-1, Prolactin, SCC and Her2/Neu and, optionally, also of any one or more of CA125, CEA, CRP, NSE and C4; or (ii) CYFRA 21-1, CEA and IL-6; or (iii) CYFRA 21-1, SCC, NSE, TIMP-1 and Her2/Neu in a subject's sample and in a control sample, it may be determined whether a change or alteration of the level of expression is given. Depending on the nature of the control sample, it may be deduced, whether the subject is affected by a lung cancer disease or condition as mentioned herein, e.g. lung cancer, a lung cancer disease showing cancerous lung nodules, NSCLC or SCLC. For example, in case the control sample is a sample of a healthy subject, any changed level of expression of (i) CYFRA 21-1, Prolactin, SCC and Her2/Neu and, optionally, also of any one or more of CA125, CEA, CRP, NSE and C4; or (ii) CYFRA 21-1, CEA and IL-6; or (iii) CYFRA 21-1, SCC, NSE, TIMP-1 and Her2/Neu in the test sample in comparison to the control sample is considered to be indicative of a lung cancer disease. In case the control sample is a sample of a cancerous subject, e.g. a lung cancer subject, a similar or identical level of expression of (i) CYFRA 21-1, Prolactin, SCC and Her2/Neu and, optionally, also of any one or more of CA125, CEA, CRP, NSE and C4; or (ii) CYFRA 21-1, CEA and IL-6; or (iii) CYFRA 21-1, SCC, NSE, TIMP-1 and Her2/Ne, in both samples is considered to be indicative of a lung cancer disease or condition as mentioned herein, e.g. lung cancer, a lung cancer disease showing cancerous lung nodules, NSCLC or SCLC.

The term "changed" in the context of the present invention thus denotes an increase or decrease in the expression level between a situation to be analyzed, e.g. derivable from a subject's sample, and a reference point. Expression levels are deemed to be "changed" when an expression level, e.g. in a subject's sample to be analyzed, differs by, i.e. is elevated or reduced by, for example, 5%, 6%, 7%, 8%, 9%, 10%, 15%, 20%, 25%, 30%, 40%, 50%, or more than 50% in comparison to a healthy control level, or is elevated at least 0.1 fold, at least 0.2 fold, at least 1 fold, at least 2 fold, at least 5 fold, or at least 10 fold or more in comparison to a healthy control level. If a comparison with a cancerous control level is to be carried out, an additional comparison with a healthy control level may be performed. Such an additional comparison allows for the determination of a tendency of the modification, e.g. the magnitude of an increase or decrease of the expression level may be observed and/or corresponding conclusions may be drawn.

If the expression levels of more than one biomarker are determined, e.g. of (i) CYFRA 21-1, Prolactin, SCC and Her2/Neu and, optionally, also any one or more of CA125, CEA, CRP, NSE and C4; or of (ii) CYFRA 21-1, CEA and IL-6; or of (iii) CYFRA 21-1, SCC, NSE, TIMP-1 and Her2/Neu, the obtained data may be statistically analyzed. For example, a measured change of expression levels may be averaged or weighed according to a suitable procedure in order to generate a summary value for the change. It is preferred that the contributions to said summary value are identical for all measured biomarkers.

In further preferred embodiments, the determination of the levels of expression of (i) CYFRA 21-1, Prolactin, SCC and Her2/Neu and, optionally, also of any one or more of CA125, CEA, CRP, NSE and C4; or of (ii) CYFRA 21-1, CEA and IL-6; or of (iii) CYFRA 21-1, SCC, NSE, TIMP-1 and Her2/Neu in a subject's sample is performed in an automatic analyzer. Particularly preferred is the employment of an integrated immunoassay and clinical chemistry analyzers.

In a further specific embodiment, the present invention relates to corresponding methods (i) for detecting a lung cancer disease, (ii) discriminating a non-cancerous lung disease showing benign pulmonary nodules from a lung cancer disease showing cancerous lung nodules, or (iii) discriminating lung cancer subtypes NSCLC and SCLC. The methods may comprise, in a further specific embodiment, a step of determining the level of expression of a reference marker in the subject's sample; or determining the level of an internal standard. The term "reference marker" as used herein refers to any suitable biomarker, e.g. to a steadily expressed and continuously detectable expression product, protein or protein variant in the organism of choice. The term "internal standard" as used herein refers to a protein or other type of marker, which is typically used in automatic analyzers in order to calibrate the amount, e.g. concentration of expression products or proteins. Similarly, also the internal standard may, in typical embodiments, be correlated to a corresponding amount, e.g. concentration, of biomarkers according to the present invention which may have been determined before, e.g. in cancer or non-cancer situations, or be known to the skilled person, e.g. from databases, suitable literature sources etc.

By determining the amount of a reference marker or an internal standard, a calibration of the determined levels of expression becomes possible. Accordingly, in certain embodiments, the measured expression levels of (i) CYFRA 21-1, Prolactin, SCC and Her2/Neu and, optionally, also of any one or more of CA125, CEA, CRP, NSE and C4; or (ii) CYFRA 21-1, CEA and IL-6; or (iii) CYFRA 21-1, SCC, NSE, TIMP-1 and Her2/Neu may be normalized to the expression of the reference marker or internal standard. The normalization may be performed according to any suitable method known to the person skilled in the art, e.g. according to normalization statistical methods like the standard z-score, Student's T-statistic, studentized residual analysis, standardized moment statistic, or coefficient variation statistic. Further envisaged are algorithmic approaches, which allow for a suitable internal comparison, being equivalent to a calibration approach, as will be explained further below.

In a further aspect A, the present invention relates to a computer-implemented method for providing a trained machine learning model for the analysis of a sample, comprising the steps: (a) obtaining data on the levels of expression of a group of biomarkers comprising at least CYFRA 21-1, Prolactin, SCC and Her2/Neu and, optionally, also any one or more of CA125, CEA, CRP, NSE and C4, from a cohort of lung cancer patients and healthy subjects from a data storage; (b) performing a machine learning method such as a logistic regression, LDA, Random Forest analysis, XGBoost analysis, decision tree analysis or SVM using the data on the levels of expression of CYFRA 21-1, Prolactin, SCC and Her2/Neu and, optionally also of any one or more of CA125, CEA, CRP, NSE and C4, of the cohort of step (a); and (c) training a machine learning model with the result obtained in step (b) and with diagnostic, prognostic and/or predictive conclusions in the context of lung cancer corresponding to said expression, thereby obtaining a trained machine learning model.

In a similar aspect B, the present invention relates to a computer-implemented method for providing a trained machine learning model for the analysis of a sample, comprising the steps: (a) obtaining data on the levels of expression of a group of biomarkers comprising at least CYFRA 21-1, CEA and IL-6 from a cohort of patients with a non-cancerous lung disease showing benign pulmonary nodules and of patients with a lung cancer disease showing cancerous lung nodules from a data storage; (b) performing a machine learning method such as a logistic regression, LDA, Random Forest analysis, XGBoost analysis, decision tree analysis or SVM using the levels of expression of CYFRA 21-1, CEA and IL-6 of the cohort of step (a); and (c) training a machine learning model with the result obtained in step (b) and with diagnostic, prognostic and/or predictive conclusions in the context of lung cancer disease or non-cancerous lung disease corresponding to said expression, thereby obtaining a trained machine learning model.

In a similar aspect C, the present invention relates to a computer-implemented method for providing a trained machine learning model for the analysis of a sample comprising the steps: (a) obtaining data on the levels of expression of a group of biomarkers comprising at least CYFRA 21-1, SCC, NSE, TIMP-1 and Her2/Neu from a cohort of patients with lung cancer subtype NSCLC and of patients with cancer subtype SCLC from a data storage; (b) performing a machine learning method such as a logistic regression, LDA, Random Forest analysis, XGBoost analysis, decision tree analysis or SVM using the levels of expression of CYFRA 21-1, SCC, NSE, TIMP-1 and Her2/Neu of the cohort of step (a); and (c) training a machine learning model with the result obtained in step (b) and with diagnostic, prognostic and/or predictive conclusions in the context of lung cancer subtypes NSCLC or SCLC corresponding to said expression, thereby obtaining a trained machine learning model.

The method thus starts with a step of data collection on the levels of expression of (i) CYFRA 21-1, Prolactin, SCC and Her2/Neu and, optionally, also of any one or more of CA125, CEA, CRP, NSE and C4, of the samples of a cohort of lung cancer patients and healthy subjects; (ii) CYFRA 21-1, CEA and IL-6 of the samples of a cohort of patients with a non-cancerous lung disease showing benign pulmonary entities and of patients with a lung cancer disease showing cancerous lung nodules; or (iii) CYFRA 21-1, SCC, NSE, TIMP-1 and Her2/Neu of the samples of a cohort patients with lung cancer subtype NSCLC and of patients with cancer subtype SCLC.

The term "data on the level of expression" as used herein refers to a set of data which defines the levels of expression as defined herein, e.g. concentrations of proteins (i) CYFRA 21-1, Prolactin, SCC and Her2/Neu and, optionally, also any one or more of CA125, CEA, CRP, NSE and C4; (ii) CYFRA 21-1, CEA and IL-6; or (iii) CYFRA 21-1, SCC, NSE, TIMP-1 and Her2/Neu.

In specific embodiments, the data on the levels of expression may alternatively be derived from an expression data database, e.g., a publicly available database or from literature repositories with database functions. For example, the data may be derived from the Human Protein Atlas (https://www.proteinatlas.org/), or the OncoDB (https://oncodb.org/) .

The data is obtained in any suitable text-based format, e.g., as CSV or XML.

The data may be stored locally or in a cloud system or may be derived from the data storage or database on-the-fly. The step of obtaining data may, in certain embodiments, also include an update functionality which searches for changes to level of expression data in the database. This update may, for example, be performed within a predefined period, e.g., every week, 2, 3, 4 weeks, 2 months, 3, months, 4 months, etc.

The data may, in specific embodiments, be modified or processed, e.g., transformed into a new format, analyzed with respect to a feature of interest, or combined in any suitable way.

For aspect A, i.e. the detection of lung cancer, the expression data is obtained from at least two groups of persons, a group of lung cancer patients and a group of healthy subjects. The lung cancer patients may preferably comprise patients affected by different types, subtypes or stages of cancer, e.g. lung cancer stage I, II or III. In alternative embodiments, the group may also comprise patients affected by the same type, subtype or stage of lung cancer. Also envisaged are groups of lung cancer patients being affected by different cancer types. A very good performance of the model can be expected if the group of lung cancer patients and healthy subjects is of similar size. Further envisaged are groups which alternatively or additionally comprise patients which are comparable or similar with respect to further characteristics such as distribution of age, sex, smoking status (former or current smoker), smoking intensity, smoking duration, duration of quitting smoking, ethnicity, personal history of cancer, familial cancer predisposition, Body Mass Index, education, or other presence of lung diseases like COPD.

For aspect B, i.e. the discrimination of a non-cancerous lung disease showing benign pulmonary nodules from a lung cancer disease showing cancerous lung nodules, the expression data is obtained from at least two groups of persons, a group of patients with a non-cancerous lung disease showing benign pulmonary nodules and a group of patients with a lung cancer disease showing cancerous lung nodules. The cohort of patients with a non-cancerous lung disease showing benign pulmonary nodules may preferably comprise patients with pulmonary nodules, which have been confirmed to be benign. The cohort may comprise patients showing different types of non-cancerous lung diseases displaying benign pulmonary nodules, e.g. fungal infections, bacterial infections, hamartomas etc. or patients affected by different stages of these diseases. The cohort of patients with a lung cancer disease showing cancerous lung nodules may preferably comprise patients with pulmonary nodules, which have been confirmed to be cancerous. The cohort may, in certain embodiments, further comprise different types, subtypes or stages of lung cancer showing cancerous lung nodules, preferably determined by histological examination after biopsy or surgical tissue removal. In alternative embodiments, the cohort may also comprise patients affected by the same type, subtype or stage of lung cancer showing cancerous lung nodules. Also envisaged are cohorts of lung cancer patients being affected by different cancer types showing cancerous lung nodules. A very good performance of the model can be expected if the cohorts of patients with a lung cancer disease showing cancerous lung nodules and patients with a non-cancerous lung disease showing benign pulmonary nodules are of similar size. Further envisaged are cohorts which alternatively or additionally comprise patients which are comparable or similar with respect to further characteristics such as distribution of age, sex, smoking status (former or current smoker), smoking intensity, smoking duration, duration of quitting smoking, ethnicity, personal history of cancer, familial cancer predisposition, Body Mass Index, education, or other presence of lung diseases like COPD.

For aspect C, i.e. the discrimination of the lung cancer subtypes NSCLC and SCLC, the expression data is obtained from at least two groups of persons, a group of patients with lung cancer subtype NSCLC and a group of patients with cancer subtype SCLC. The cohort of patients with lung cancer subtype NSCLC may preferably comprise patients with a histologically confirmed NSCLC diagnosis. The cohort may comprise patients affected by different types, subtypes or stages of NSCLC, or it may comprise patients affected by the same types, subtypes or stages of NSCLC. The cohort of patients with lung cancer subtype SCLC may preferably comprise patients with a histologically confirmed SCLC diagnosis. The cohort may comprise patients affected by different types, subtypes or stages of SCLC, or it may comprise patients affected by the same types, subtypes or stages of SCLC. A very good performance of the model can be expected if the cohorts of patients with NSCLC and patients with a SCLC are of similar size. Further envisaged are cohorts which alternatively or additionally comprise patients which are comparable or similar with respect to further characteristics such as distribution of age, sex, smoking status (former or current smoker), smoking intensity, smoking duration, duration of quitting smoking, ethnicity, personal history of cancer, familial cancer predisposition, Body Mass Index, education, or other presence of lung diseases like COPD.

In a subsequent step, a machine learning method is performed. The machine learning method is, in preferred embodiments, a linear combination of the levels of expression of (i) CYFRA 21-1, Prolactin, SCC and Her2/Neu and, optionally, also of any one or more of CA125, CEA, CRP, NSE and C4; or (ii) CYFRA 21-1, CEA and IL-6; or (iii) CYFRA 21-1, SCC, NSE, TIMP-1 and Her2/Neu.

The term "machine learning method" as used herein refers to a any suitable method which implements an analysis of the levels of expression as defined above. In preferred embodiments, the machine learning method is a logistic regression, LDA, Random Forest analysis, XGBoost analysis, decision tree analysis or support vector machine. It is particularly preferred that the machine learning method is a logistic regression.

In particularly preferred embodiments, the logistic regression algorithm for biomarker group (i) CYFRA 21-1, Prolactin, SCC and Her2/Neu and, optionally also any one or more of CA125, CEA, CRP, NSE and C4, is based on the following procedure: Raw assay data are log-transformed, followed by computing the mean and standard deviation for all the samples. The data is then standardized so that samples have a mean of 0 and a standard deviation of 1. Thereafter a regression model as follows is applied:

$$X = a_{CYFRA\ 21\text{-}1} \cdot \log(C_{CYFRA\ 21\text{-}1}) + a_{Prolactin} \cdot \log(C_{Prolactin}) + a_{SCC} \cdot \log(C_{SCC}) + a_{Her2/Neu} \cdot \log(C_{Her2/Neu}) + a_0$$

or, optionally,

$$X = a_{CYFRA\ 21\text{-}1} \cdot \log(C_{CYFRA\ 21\text{-}1}) + a_{Prolactin} \cdot \log(C_{Prolactin}) + a_{SCC} \cdot \log(C_{SCC}) + a_{Her2/Neu} \cdot \log(C_{Her2/Neu}) + a_{CA125} \cdot \log(C_{CA125}) + a_{CEA} \cdot \log(C_{CEA}) + a_{CRP} \cdot \log(C_{CRP}) + a_{NSE} \cdot \log(C_{NSE}) + a_{C4} \cdot \log(C_{C4}) + a_0,$$

wherein $a_i$ is the to-be-learned coefficient for each marker amount or concentration $C_i$ and $a_0$ is a standard bias term. The determination of said values $C_i$ has been described herein above in the context of the assay methods of the present invention. The linear combination X of the measured expression data, preferably concentrations, is fed into the logistic function to obtain a value between 0 and 1. Using a suitable threshold the samples can be classified as tumor vs. non-tumor. In particular, the overall value of X is of importance. The coefficients $a_i$ may be determined, for example, by using a training set, e.g. a subset of the sample group, which is only used for learning those coefficients such that the classification error is small within the training set. The performance metrics may then be estimated on an independent test sample set.

In particularly preferred embodiments, the logistic regression algorithm for biomarker group (ii) CYFRA 21-1, CEA and IL-6 is based on the following procedure: Raw assay data are log-transformed, followed by computing the mean and standard deviation for all the samples. The data is then standardized so that samples have a mean of 0 and a standard deviation of 1. Thereafter a regression model as follows is applied:

$$X = a_{CYFRA\ 21-1} \cdot \log(C_{CYFRA\ 21-1})\ a_{CEA} \cdot \log(C_{CEA}) + a_{IL-6} \cdot \log(C_{IL-6}) + a_0,$$

wherein $a_i$ is the to-be-learned coefficient for each marker amount or concentration $C_i$ and $a_0$ is a standard bias term. The determination of said values $C_i$ has been described herein above in the context of the assay methods of the present invention. The linear combination X of the measured expression data, preferably concentrations, is fed into the logistic function to obtain a value between 0 and 1. Using a suitable threshold the samples can be classified as non-cancerous lung disease showing benign pulmonary nodules vs. a lung cancer disease showing cancerous lung nodules. In particular, the overall value of $X$ is of importance. The coefficients $a_i$ may be determined, for example, by using a training set, e.g. a subset of the sample group, which is only used for learning those coefficients such that the classification error is small within the training set. The performance metrics may then be estimated on an independent test sample set.

In further particularly preferred embodiments, the diagnostic algorithm for biomarker group (iii) CYFRA 21-1, SCC, NSE, TIMP-1 and Her2/Neu is based on a decision tree. The decision tree node cutoffs may be determined by a Brute-Force search over all possible decision trees using a training set, e.g., a subset of the sample cohort.

Logistic regression is a supervised machine learning algorithm that accomplishes binary classification tasks by predicting the probability of an outcome, event, or observation. The algorithm analyzes the relationship between one or more independent variables and classifies data into discrete classes. It is typically used in predictive modeling, where the model estimates the mathematical probability of whether an instance belongs to a specific category or not. According to the present invention the linear combination X with the trained coefficients $a_i$ (and $a_0$) can be computed for newly measured expression data, in particular concentrations $C_i$ and then fed into the logistic function to obtain a value between 0 and 1, which can be interpreted as the probability of the sample being a cancer. Choosing a classification threshold for this probability (e.g. by computing the Youden Index (Youden, 1950, Cancer, Vol. 3, Issue 1, 32-35) on the corresponding Receiver Operating Characteristic Curve allows for a final classification in tumor vs. non-tumor, or non-cancerous lung disease showing benign pulmonary nodules vs. a lung cancer disease showing cancerous lung nodules.

The linear discriminant analysis (LDA) is a technique for dimensionality reduction problems as pre-processing step for pattern classification applications. The main purpose of the algorithm is the reduction of dimensions by removing redundant and dependent features from a higher dimensional space to a space with lower dimensions. Typically, LDA is used in the context of the present invention in classifier mode. The algorithm may accordingly be used to distinguish between two or more groups that are described by different variables. Further information can be derived from suitable literature sources such as Petros Xanthopoulos, Panos M. Pardalos & Theodore B. Trafalis, Robust Data Mining, Springer New York, NY, 2012, Chapter: Linear Discriminant Analysis, p27-33.

Random forest analysis is an ensemble learning method for classification, regression and other tasks that operates by constructing a multitude of decision trees at training time. For classification tasks, the output of the random forest is the class selected by most trees. For regression tasks, the mean or average prediction of the individual trees is returned. Random forests correct for decision trees' habit of overfitting to their training set. Further information can be derived from suitable literature sources such as L. Breiman, 2001, Machine Learning, Vol. 45, 5-32.

XGBoost, or Extreme Gradient Boosting, is a scalable, distributed gradient-boosted decision tree (GBDT) machine learning library. It provides parallel tree boosting and is a machine learning library for regression, classification, and ranking problems. GBDT is a decision tree ensemble learning algorithm similar to random forest, for classification and regression. A model is typically build consisting of multiple decision trees. Further information can be derived from suitable literature sources such as Chen et al., 2016, Proceedings of the 22nd ACM SIGKDD International Conference on Knowledge Discovery and Data Mining, 785- 794.

Decision tree analysis is a process of drawing a decision tree, i.e. a graphic representation of alternative solutions that are available to solve a given problem, in order to determine the most effective courses of action. Decision trees are comprised of nodes and branches wherein nodes represent a test on an attribute and branches represent potential alternative outcomes. Further information can be derived from suitable literature sources such as Jiao et al., 2020, J. Phys.: Conf. Ser, 1651, 012083.

A support vector machine (SVM) is a machine learning algorithm that uses supervised learning models to solve complex classification, regression, and outlier detection problems by performing optimal data transformations that determine boundaries between data points based on predefined classes, labels, or outputs. Further information can be derived from suitable literature sources such as C. Cortes, V. Vapnik, 1995, Machine Learning, Vol. 20, 273-29.

In a subsequent step of the methods for providing trained machine learning models, the machine learning models are trained based on the result obtained in previous steps (b) and diagnostic, prognostic and/or predictive conclusions in the context of (i) lung cancer corresponding to said expression, thereby obtaining a trained machine learning model; or (ii) a non-cancerous lung disease showing benign pulmonary nodules and a lung cancer disease showing cancerous lung nodules corresponding to said expression, thereby obtaining a trained machine learning model; or (iii) lung cancer subtypes NSCLC or SCLC corresponding to said expression, thereby obtaining a trained machine learning model.

Further, by using a known health status associated to a certain sample during training allows to optimize the parameters/coefficients of the model. In general, the machine learning methods are based on the strategy to train and optimize a machine learning model and its coefficients/parameters based on a training set and then assess the performance of the machine learning model based on a test set.

The term "diagnostic, prognostic and/or predictive conclusions" as used herein refers to information on medical or therapeutic consequences of certain data on the levels of expression of (i) CYFRA 21-1, Prolactin, SCC and Her2/Neu and, optionally, also of any one or more of CA125, CEA, CRP, NSE and C4; or (ii) CYFRA 21-1, CEA and IL-6; or (iii) CYFRA 21-1, SCC, NSE, TIMP-1 and Her2/Neu. For example, if a certain level of expression which is found in the data of one group, e.g. of lung cancer patients, or NSCLC (but not in the data of the group of healthy patients, or SCLC) is connected, for example, to a lung cancer disease or NSCLC, this connection or link may be translated into a diagnostic output, e.g., a classification as in terms of disease, disease subtype etc. Corresponding information may, for example, be derived from suitable literature sources or database entries associated with the level of expression data, but can also be derived purely based on training of the machine learning method on an annotated cohort. Similarly, if certain levels of expression are connected to cancerous pulmonary nodules of a lung cancer disease, this connection or link may be translated into a prognostic output. Corresponding information may, for example, be derived from suitable literature sources or database entries associated with the associated level of expression data. Also, should certain level of expression patterns be connected to therapeutic suggestions or instructions in the context of a lung cancer disease, pulmonary nodules, cancer subtypes etc., this connection or link may be translated into a predictive output, e.g., reflected by a corresponding classification. Corresponding information may, for example, be derived from suitable literature sources or database entries associated with the level of expression data.

In particularly preferred embodiments, the ground truth data for the above mentioned machine learning approaches comprises at least of 100 datasets, more preferably at least 1,000 datasets, most preferably at least 10,000 datasets, wherein a dataset comprises at least a group of biomarkers measured in a sample as described herein and ground truth annotations of said sample.

In a further aspect D, the present invention relates to a computer-implemented method for the analysis of a sample of a subject comprising the steps: (a) obtaining data on the levels of expression of a group of biomarkers comprising CYFRA 21-1, Prolactin, SCC and Her2/Neu and, optionally, also of any one or more of CA125, CEA, CRP, NSE and C4 in the subject's sample, preferably from a data storage; (b) inputting the obtained expression data of step (a) into a trained machine learning model, wherein the machine learning model is based on a machine learning method such as a logistic regression, LDA, Random Forest analysis, XGBoost analysis, decision tree analysis or SVM using the expression data, wherein the machine learning model is trained based on a training cohort of lung cancer patients and healthy subjects and corresponding diagnostic, prognostic and/or predictive conclusions in the context of lung cancer disease; and (c) deriving a diagnostic, prognostic and/or predictive conclusion output with respect to the detection of the lung cancer disease in the subject.

In a further similar aspect E, the present invention relates to a computer-implemented method for the analysis of a sample of a subject comprising the steps: (a) obtaining data on the levels of expression of a group of biomarkers comprising CYFRA 21-1, CEA and IL-6 in the subject's sample, preferably from a data storage; (b) inputting the obtained expression data of step (a) into a trained machine learning model, wherein the machine learning model is based on a machine learning method such as a logistic regression, LDA, Random Forest analysis, decision tree analysis, XGBoost analysis, decision tree analysis or SVM using the expression data, and wherein the machine learning model is trained based on a training cohort of patients with a non-cancerous lung disease showing benign pulmonary nodules and a cohort of patients with a lung cancer disease showing cancerous lung nodules and corresponding diagnostic, prognostic and/or predictive conclusions in the context of lung cancer disease or non-cancerous lung disease; and (c) deriving a diagnostic, prognostic and/or predictive conclusion output with respect to the detection of the lung cancer disease or non-cancerous lung disease in the subject.

In a further similar aspect F, the present invention relates to a computer-implemented method for the analysis of a sample of a subject comprising the steps: (a) obtaining data on the levels of expression of a group of biomarkers comprising CYFRA 21-1, SCC, NSE, TIMP-1 and Her2/Neu in the subject's sample, preferably from a data storage; (b) inputting the obtained expression data of step (a) into a trained machine learning model, wherein the machine learning model is based on a machine learning method such as a logistic regression, LDA, Random Forest analysis, decision tree analysis, XGBoost analysis, decision tree analysis or SVM using the expression data, and wherein the machine learning model is trained based on a training cohort of patients with lung cancer subtypes NSCLC and of patients with SCLC and corresponding diagnostic, prognostic and/or predictive conclusions in the context of NSCLC disease or SCLC disease; and (c) deriving a diagnostic, prognostic and/or predictive conclusion output with respect to the detection of the lung cancer subtypes NSCLC or SCLC in the subject.

Step (a) of the above outlined methods essentially corresponds to the step of obtaining levels of expression data of the sample of a target subject or patient from a data storage as defined herein above. Further the trained machine learning model is a model as described herein above in detail. Accordingly, a trained machine learning model as described herein is used for the inputting step of this method. Similarly, the step (c) of the method essentially corresponds to the inference step as described in detail herein.

In a further embodiment, the present invention relates to a computer-implemented method for the analysis of a sample of a subject comprising the steps: (a) obtaining data on the levels of expression of a group of biomarkers comprising at least CYFRA 21-1, Prolactin, SCC and Her2/Neu and, optionally, also any one or more of CA125, CEA, CRP, NSE and C4, from a cohort of lung cancer patients and healthy subjects from a data storage; (b) performing a machine learning method such as a logistic regression, LDA, Random Forest analysis, XGBoost analysis, decision tree analysis or SVM using the levels of expression of CYFRA 21-1, Prolactin, SCC and Her2/Neu and, optionally, also of any one or more of CA125, CEA, CRP, NSE and C4, of the cohorts of step (a); (c) training a machine learning model with the result obtained in step (b) and diagnostic, prognostic and/or predictive conclusions in the context of lung cancer corresponding to said expression, thereby obtaining a trained machine learning model; (d) obtaining data on the levels of expression of CYFRA 21-1, Prolactin, SCC and Her2/Neu and, optionally, also of any one or more of CA125, CEA, CRP, NSE and C4, from a subject's sample, preferably from a data storage; (e) inputting the obtained expression data of step (d) into the trained machine learning model of step (c); and (f) deriving a diagnostic, prognostic and/or predictive conclusion output with respect to the detection of a lung cancer disease in the subject.

In a further similar embodiment, the present invention relates to a computer-implemented method for the analysis of a sample of a subject comprising the steps: (a) obtaining data on the levels of expression of a group of biomarkers comprising at least CYFRA 21-1, CEA and IL-6 from a cohort of patients with a non-cancerous lung disease showing benign pulmonary nodules and a cohort of patients with a lung cancer disease showing cancerous lung nodules from a data storage; (b) performing a machine learning method such as a logistic regression, LDA, Random Forest analysis, XGBoost analysis, decision tree analysis or SVM using the levels of expression of CYFRA 21-1, CEA and IL-6 of the cohorts of step (a); (c) training a machine learning model with the result obtained in step (b) and diagnostic, prognostic and/or predictive conclusions in the context of lung cancer disease or non-cancerous lung disease corresponding to said expression, thereby obtaining a trained machine learning model; (d) obtaining data on the level of expression of a group of biomarkers comprising CYFRA 21-1, CEA and IL-6 of the subject's sample, preferably from a data storage;(e) inputting the obtained expression data of step (d) into the trained machine learning model of step (c); and (f) deriving a diagnostic, prognostic and/or predictive conclusion output with respect to the detection of the lung cancer disease or non-cancerous lung disease in the subject.

In a yet another similar embodiment, the present invention relates to a computer-implemented method for the analysis of a sample of a subject comprising the steps: (a) obtaining data on the levels of expression of a group of biomarkers comprising CYFRA 21-1, SCC, NSE, TIMP-1 and Her2/Neu from a cohort of patients with lung cancer subtype NSCLC and a cohort of patients with cancer subtype SCLC from a data storage; (b) performing a machine learning method such as a logistic regression, LDA, Random Forest analysis, XGBoost analysis, decision tree analysis or SVM using the levels of expression of CYFRA 21-1, SCC, NSE, TIMP-1 and Her2/Neu of the cohorts of step (a); (c) training a machine learning model with the result obtained in step (b) and with diagnostic, prognostic and/or predictive conclusions in the context of lung cancer subtypes NSCLC or SCLC corresponding to said expression, thereby obtaining a trained machine learning model; (d) obtaining data on the levels of expression of a group of biomarkers comprising CYFRA 21-1, SCC, NSE, TIMP-1 and Her2/Neu of the subject's sample, preferably from a data storage;(e) inputting the obtained expression data of step (d) into the trained machine learning model of step (c); and (f) deriving a diagnostic, prognostic and/or predictive conclusion output with respect to the detection of the lung cancer subtypes NSCLC or SCLC in the subject.

The steps of these "combined" methods essentially correspond to steps as defined in the computer-implemented methods for the analysis of a sample of a subject and the computer-implemented methods for providing a trained machine learning model for the analysis of a sample as defined herein.

In another aspect, the present invention relates to a data processing device comprising means for carrying out the computer-implemented methods as defined above. The device comprises means for carrying out any one or more steps of the computer-implemented methods of the present invention as mentioned herein above. Accordingly, any of the computer-implemented methods described herein may be totally or partially performed with a computer system including one or more processor (s), which can be configured to perform the steps. Accordingly, some of the present embodiments are directed to computer systems configured to perform the steps of any of the computer-implemented methods described herein, potentially with different components performing respective steps or a respective group of steps. Corresponding steps of methods may further be performed at the same time or in a different order. Additionally, portions of these steps may be used with portions of other steps from other methods. Also, all or portions of a step may be optional. Additionally, any of the steps of any of the methods can be performed with models, circuits, or other means for performing these steps.

Also envisaged is a computer program comprising instructions which, when the program is executed by a computer, cause the computer to carry out any of the computer-implemented methods of the invention as defined herein or any one or more computerizable steps of the methods of the present invention as mentioned herein.

Also envisaged is the provision of a computer-readable storage medium comprising a computer program product as defined above. The computer-readable storage medium may be connected to a server element, or be present in a cloud structure, or be connected via internet or intranet to one or more database structures, or client databases etc.

Any of the software components or computer programs or functions described herein may be implemented as software code to be executed by a processor using any suitable computer language such as, for example, Java, Python, JavaScript, VB.Net, C++, C#, C, Swift, Rust, Objective-C, Ruby, PHP, or Perl using, for example, conventional or object-oriented techniques. The software code may be stored as a series of instructions or commands on a computer readable medium for storage and/or transmission, suitable media include random access memory (RAM), a read only memory (ROM), a magnetic medium such as a hard-drive, or an optical medium such as a compact disk (CD) or DVD (digital versatile disk), flash memory, and the like. The computer readable medium may be any combination of such storage or transmission devices. Such programs may also be encoded and transmitted using carrier signals adapted for transmission via wired, optical, and/or wireless networks conforming to a variety of protocols, including the Internet. As such, a computer readable medium according to the present invention may be created using a data signal encoded with such programs. Computer readable media encoded with the program code may be packaged with a compatible device or provided separately from other devices (e.g., via internet download). Any such computer readable medium may reside on or within a single computer program product (e.g., a hard drive, a CD, or an entire computer system), and may be present on or within different computer program products within a system or network. A computer system may include a monitor, printer, or other suitable display for providing any of the results mentioned herein to a user.

In a further preferred embodiment, the training of the machine learning model as defined herein above additionally includes the inputting of data of the cohorts concerning (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests. These data may be inputted in the machine learning models at any suitable point in time. The data may be adjusted to the cohort type used. Further, for certain cohorts or individuals not all data may be available.

In a further aspect, the present invention relates to a method of detecting and assessing a lung cancer disease in a subject comprising the steps of: (i) performing the method for the analysis of a sample of a subject based on obtaining data on the levels of expression of a group of biomarkers comprising at least CYFRA 21-1, Prolactin, SCC and Her2/Neu and, optionally, also any one or more of CA125, CEA, CRP, NSE and C4, as defined herein above;

(ii) introducing the results obtained in step (i) and optionally introducing (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests into a first machine learning model as obtained in a computer-implemented method for providing a trained machine learning model based on data on the levels of expression of a group of biomarkers comprising at least CYFRA 21-1, Prolactin, SCC and Her2/Neu and, optionally, also any one or more of CA125, CEA, CRP, NSE and C4, as defined herein above;
(iii) determining with said first machine learning model a likelihood for the presence of a lung cancer disease in the subject;
(iv) performing in a subject with a high likelihood as determined in step (iii) the method for the analysis of a sample based on data on the level of expression of a group of biomarkers comprising CYFRA 21-1, CEA and IL-6 as defined herein above;
(v) introducing the results obtained in step (iv) and optionally introducing (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests into a second machine learning model as obtained in a computer-implemented method for providing a trained machine learning model based on data on the levels of expression of a group of biomarkers comprising CYFRA 21-1, CEA and IL-6 as defined herein above;

(vi) determining with said second machine learning model a likelihood for the presence of a lung cancer disease vs. a non-cancerous lung disease in the subject;

(vii) performing in a subject with a high likelihood of lung cancer disease as determined in step (vi) the method for the analysis of a sample of a subject based on data on the levels of expression of a group of biomarkers comprising CYFRA 21-1, SCC, NSE, TIMP-1 and Her2/Neu;

(viii) introducing the results obtained in step (vii) and optionally introducing (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests into a third machine learning model as obtained in a method for providing a trained machine learning model based on data on the levels of expression of a group of biomarkers comprising CYFRA 21-1, SCC, NSE, Metallopeptidase Inhibitor 1 (TIMP-1) and Her2/Neu as defined herein above; and

(ix) determining with said third machine learning model the likelihood for the presence of a lung cancer subtype NSCLC or SCLC.

In specific embodiments of said method, a subject may be diagnosed starting at step (i) or at a later step, e.g. at step (iv), if the subject has previously, e.g. with alternative methods, been determined as having a high likelihood for the presence of a lung cancer, or at step (vii) if the subject has previously, e.g. with alternative methods, been determined as having a high likelihood for the presence of a lung cancer due to the presence of cancerous pulmonary nodules. In further embodiments, certain steps may be skipped. For example, in case subject has been determined as having a high likelihood for the presence of a lung cancer in step (iii), the method may be continued with steps (vii) et seq., i.e. it may directly be tested whether the cancer is NSCLC or SCLC. In further specific embodiments, should the subject have been determined as showing pulmonary nodules, e.g. by CT or X-ray scans, the method may be started at step (iv), i.e. the subject to be tested may alternatively show a pulmonary nodule.

The term "high likelihood for the presence of" as used herein refers to the diagnostic, prognostic and/or predictive conclusion output as obtained with methods described herein, which may be provided in the form of a metric score within a clinical decision scale depending on thresholds for the expression data of the corresponding biomarker according to the present invention.

In a further embodiment, the present invention relates to the use of peptide affinity ligands for the expression products or proteins of a group of biomarkers comprising (i) at least CYFRA 21-1, Prolactin, SCC and Her2/Neu and, optionally, also any one or more of CA125, CEA, CRP, NSE and C4, for detecting a lung cancer disease. The detection may preferably be performed in a sample or in vitro.

In another embodiment, the present invention relates to the use of peptide affinity ligands for the expression products or proteins of a group of biomarkers comprising (ii) CYFRA 21-1, CEA and IL-6 for discriminating a non-cancerous lung disease from a lung cancer disease in a subject, preferably for discriminating subjects with a non-cancerous lung disease showing benign pulmonary nodules from subjects with a lung cancer disease showing cancerous lung nodules. The detection may preferably be performed in a sample or in vitro.

In yet another embodiment, the present invention relates to the use of peptide affinity ligands for the expression products or proteins of a group of biomarkers comprising (iii) CYFRA 21-1, SCC, NSE, TIMP-1 and Her2/Neu for discriminating cancer subtypes NSCLC and SCLC in a subject. The detection may preferably be performed in a sample or in vitro.

The following figures and example are provided for illustrative purposes. It is thus understood that the figures and example are not to be construed as limiting. The skilled person in the art will clearly be able to envisage further modifications of the principles laid out herein.

EXAMPLES

**Example 1**

The blood-based biomarker identification study for detecting the presence of malignant pulmonary nodules and early stages of lung cancer in individuals not known to have cancer based on significantly different in abundance of protein levels was carried out.

**1. Cohort**

In the study, 100 "normal" individuals not known to have cancer and 100 patients with lung cancer at an early stage (stage I and stage II) and at a locally advanced stage (stage III) in a gender balanced age group ranging between 48 and 86 years were selected for identifying a signature of blood-based biomarkers based on their significant abundance.

**2. Statistical analysis**

Each of the identified protein biomarkers was initially log-transformed to build a multivariate logistic regression model to distinguish individuals with lung cancer from healthy persons without cancer. The multivariate logistic regression model was trained and validated on 100 patients with lung cancer (stage I, II, III) and 100 healthy individuals using 500 repetitions of random 80-20 training-test-splits, i.e. 80 % of the data was used for training and 20 % for testing of the model.
In the logistic regression model, there were no separate individual marker thresholds, which determine the classification outcome. The quantity of importance was a linear combination of the respective feature values, e.g. the biomarker's concentrations:

$$X = \beta_0 + \beta_1 \cdot C_{PRL} + \beta_2 \cdot C_{SCC} + \cdots + \beta_9 \cdot C_{C4}$$

The coefficients β were determined by using the training data set, which was only used for learning those coefficients such that the classification error was small within the training set. The performance metrics were then estimated on an independent test sample set.

**3. Results**

A panel of 9 blood-based protein biomarkers (PRL, SCC, CYFRA21-1, NSE CA125, CEA, CRP, Her2/Neu (H2n), C4) with significantly different abundances was identified. Of 9 biomarkers, H2n and C4 had decreased levels in blood, whilst the remaining 7 biomarkers had elevated protein levels (see Figures 1, 2 and 3).
ROC analysis showed that a very high area under the curve (AUC) of 0.99 in the receiver operator curve (ROC) was achieved. Table 1 and Figure 1 show the characteristics of the classification model at the optimal operating point of the ROC determined by the maximum Youden-Index for PRL, SCC, CYFRA21-1, NSE, CA125, CEA, CRP, Her2/Neu (H2n), C4.

**Table 1:**

| Validation cohort | Lung cancer stage I, II, III | | Lung cancer stage I | | Lung cancer stage II | |
|---|---|---|---|---|---|---|
| | Mean | Std | Mean | Std | Mean | Std |
| AUC | 0.992 | 0.012 | 0.992 | 0.017 | 0.992 | 0.019 |
| Sensitivity | 0.958 | 0.047 | 0.959 | 0.061 | 0.958 | 0.062 |
| Specificity | 0.974 | 0.034 | 0.974 | 0.047 | 0.975 | 0.047 |
| PPV | 0.974 | 0.035 | 0.971 | 0.060 | 0.974 | 0.050 |
| NPV | 0.960 | 0.045 | 0.962 | 0.055 | 0.961 | 0.055 |

Performance characteristics of the multivariate logistic regression model consisting of 9 blood-based biomarkers to classify the presence of lung cancer prior to CT imaging of the chest: PRL, SCC, CYFRA21-1, NSE, CA125, CEA, CRP, Her2/Neu (H2n), C4

**Example 2**

A study on the identification of blood-based biomarkers to aid in the discrimination of benign from cancerous findings detected in CT images of the chest based on significantly different abundances of protein levels was carried out.

**1. Cohort**

In the study, 100 patients with lung cancer (stage I, II and III) and 20 patients with non-cancerous, benign lung diseases in a gender balanced age group ranging between 46 and 86 years were selected. For the benign lung diseases, cases with chronic pneumonia (n=10) or hamartoma (n=10), which exhibit similarities in appearance to lung cancer on CT images were chosen.

## 2. Statistical analysis

Based on log-transformed abundance levels of the identified protein biomarkers, a multivariate logistic regression model to distinguish individuals with lung cancer from patients with benign lung disease was built:

$$X = \beta_0 + \beta_1 \cdot C_{CYFRA21-1} + \beta_2 \cdot C_{CEA} + \beta_3 \cdot C_{IL6}$$

The multivariate logistic regression model was trained and validated on patients with lung cancer (n=20, stage I, II, III) and patients with benign lung disease (hamartoma (n=10), chronic pneumonia (n=10)) using 500 repetitions of random 80-20 training-test-splits, i.e. 80 % of the data was used for training the coefficients $\beta$ of the model and 20 % for testing of the performance of the model.

## 3. Results

A panel of 3 protein biomarkers (CYFRA21-1, CEA, IL6) with significantly increased abundance levels in the blood was identified (see Figure 4, 5 and 6).

For the 3 biomarkers model, an area under the curve (AUC) of 0.79 in the receiver operator curve (ROC) was achieved. Table 2 and Figure 2 show the characteristics of the classification model at the optimal operating point of the ROC determined by the maximum Youden-Index for CYFRA21-1, CEA, IL6.

**Table 2**

| Validation cohort | Lung cancer stage I, II, III | |
|---|---|---|
| | Mean | Std |
| AUC | 0.793 | 0.134 |
| Sensitivity | 0.616 | 0.236 |
| Specificity | 0.826 | 0.198 |
| PPV | 0.809 | 0.198 |
| NPV | 0.697 | 0.172 |

Performance characteristics of the multivariate logistic regression model consisting of 3 blood-based biomarkers to distinguish lung cancer from non-cancerous findings in the lung: CYFRA21-1, CEA, IL6.

## Example 3

A study on the identification of a diagnostic algorithm with blood-based biomarker signatures for differentiating between lung cancer subtypes prior to biopsy or surgery was carried out.

## 1. Method

In this study, patients with different types of lung cancers in a gender balanced age group ranging between 48 and 86 years were selected. Four different types of lung cancers including NSCLC adenocarcinomas (n= 47, stage I, II, III), NSCLC squamous cell carcinomas (n=31, stage I, II, III), SCLC (n= 12, stage I, II, III) and neuroendocrine SCLC (n=10, stage I, II, III) were selected analyzed.

## 2. Results

The analysis of the differences in the blood-based protein abundance levels between different lung cancer subtypes showed trends below the exploratory threshold of p<0.1 for the biomarkers CYFRA21-1, SCC, NSE, TIMP-1, and Her2/Neu (H2n). SCLC exhibited a significantly (p<0.05) lower level of H2n in comparison to the other lung cancer subtypes, whilst CYFRA21-1 was significantly (p<0.05) lower in abundance in NSCLC squamous cell carcinomas (See Figure 7).

Wide ranges of the parameter space of protein abundance level thresholds in a Brute-Force approach were tested to find suitable thresholds for lung cancer subtype discrimination (NSCLC vs SCLC).

The following protein markers were considered for building up a model algorithm: Her2/Neu (H2n), CYFRA21-1, SCC, TIMP-1, and NSE. With these biomarkers, a basic structure of a diagnostic algorithm similar to a decision tree is shown in Figure 9. In Figure 8 the Sensitivities/False Positive Rates for various threshold combinations were plotted. The optimal performance and therefore optimal threshold combination was determined by the largest distance between the diagonal "random guess line" and any point of the combination of the false positive rate and the sensitivity. The optimal threshold values were given in Figure 9 and a Sensitivity of 0.76 and a Specificity of 0.77 were yielded on our data set (Specificity was defined as (1- False Positive Rate)).

## Claims

1.  A composition for detecting a lung cancer disease, comprising a peptide affinity ligand for a protein of each biomarker of a group of biomarkers, the group of biomarkers comprising at least CYFRA 21-1, Prolactin, Squamous Cell Carcinoma Antigen (SCC) and Her2/Neu and, preferably, additionally comprising a peptide affinity ligand for a protein of one or more of Cancer antigen 125 (CA125), carcinoembryonic antigen (CEA), C-reactive protein (CRP), Neuron Specific Enolase (NSE) and C4 complement (C4).

2.  A composition for discriminating a non-cancerous lung disease showing benign pulmonary nodules from a lung cancer disease showing cancerous lung nodules, comprising a peptide affinity ligand for a protein of each biomarker of a group of biomarkers, the group of biomarkers comprising CYFRA 21-1, CEA and Interleukin 6 (IL-6).

3.  A composition for discriminating lung cancer subtypes Non-small-cell lung cancer (NSCLC) and small-cell lung cancer (SCLC), comprising a peptide affinity ligand for a protein of each biomarker of a group of biomarkers, the group of biomarkers comprising CYFRA 21-1, SCC, NSE, Metallopeptidase Inhibitor 1 (TIMP-1) and Her2/Neu.

4.  A method of detecting a lung cancer disease in a subject, comprising at least the step of determining the levels of expression of CYFRA 21-1, Prolactin, SCC and Her2/Neu and, optionally, also of any one or more of CA125, CEA, CRP, NSE and C4 in a sample of the subject.

5.  A method of discriminating a non-cancerous lung disease from a lung cancer disease in a subject, comprising at least the step of determining the levels of expression of CYFRA 21-1, CEA and IL-6 in a sample of the subject.

6.  A method of discriminating lung cancer subtypes NSCLC and SCLC in a subject, comprising at least the step of determining the levels of expression of CYFRA 21-1, SCC, NSE, TIMP-1 and Her2/Neu in a sample of the subject.

7.  A computer-implemented method for the analysis of a sample of a subject, comprising the steps:

    (a) obtaining data on the levels of expression of a group of biomarkers comprising at least CYFRA 21-1, Prolactin, SCC and Her2/Neu and, optionally, also any one or more of CA125, CEA, CRP, NSE and C4, in the subject's sample, preferably from a data storage;
    (b) inputting the obtained expression data of step (a) into a trained machine learning model

        - wherein the machine learning model is based on a machine learning method such as a logistic regression, LDA, Random Forest analysis, XGBoost analysis, decision tree analysis or SVM using the expression data,
        - wherein the machine learning model is trained based on a training cohort of lung cancer patients and healthy subjects and corresponding diagnostic, prognostic and/or predictive conclusions in the context of lung cancer disease; and

    (c) deriving a diagnostic, prognostic and/or predictive conclusion output with respect to the detection of the lung cancer disease in the subject.

8.  A computer-implemented method for providing a trained machine learning model for the analysis of a sample, comprising the steps:

    (a) obtaining data on the levels of expression of a group of biomarkers comprising at least CYFRA 21-1, Prolactin, SCC and Her2/Neu and, optionally, also any one or more of CA125, CEA, CRP, NSE and C4, from a cohort of lung cancer patients and healthy subjects from a data storage;

(b) performing a machine learning method such as a logistic regression, LDA, Random Forest analysis, XGBoost analysis, decision tree analysis or SVM using the levels of expression of CYFRA 21-1, Prolactin, SCC and Her2/Neu and, optionally also of any one or more of CA125, CEA, CRP, NSE and C4 of the cohorts of step (a);

(c) training a machine learning model with the result obtained in step (b) and with diagnostic, prognostic and/or predictive conclusions in the context of lung cancer corresponding to said expression, thereby obtaining a trained machine learning model.

9. A computer-implemented method for the analysis of a sample of a subject, comprising the steps:

(a) obtaining data on the levels of expression of a group of biomarkers comprising CYFRA 21-1, CEA and IL-6 in the subject's sample, preferably from a data storage;

(b) inputting the obtained expression data of step (a) into a trained machine learning model

- wherein the machine learning model is based on a machine learning method such as a logistic regression, LDA, Random Forest analysis, decision tree analysis, XGBoost analysis, decision tree analysis or SVM using the expression data,
- wherein the machine learning model is trained based on a training cohort of patients with a non-cancerous lung disease showing benign pulmonary nodules and of patients with a lung cancer disease showing cancerous lung nodules and with corresponding diagnostic, prognostic and/or predictive conclusions in the context of lung cancer disease or non-cancerous lung disease; and

(c) deriving a diagnostic, prognostic and/or predictive conclusion output with respect to the detection of the lung cancer disease or non-cancerous lung disease in the subject.

10. A computer-implemented method for providing a trained machine learning model for the analysis of a sample, comprising the steps:

(a) obtaining data on the levels of expression of a group of biomarkers comprising CYFRA 21-1, CEA and IL-6 from a cohort of patients with a non-cancerous lung disease showing benign pulmonary nodules and of patients with a lung cancer disease showing cancerous lung nodules from a data storage;

(b) performing a machine learning method such as a logistic regression, LDA, Random Forest analysis, XGBoost analysis, decision tree analysis or SVM using the levels of expression of CYFRA 21-1, CEA and IL-6 of the cohorts of step (a);

(c) training a machine learning model with the result obtained in step (b) and with diagnostic, prognostic and/or predictive conclusions in the context of lung cancer disease or non-cancerous lung disease corresponding to said expression, thereby obtaining a trained machine learning model.

11. A computer-implemented method for the analysis of a sample of a subject, comprising the steps:

(a) obtaining data on the levels of expression of a group of biomarkers comprising CYFRA 21-1, SCC, NSE, TIMP-1 and Her2/Neu in the subject's sample, preferably from a data storage;

(b) inputting the obtained expression data of step (a) into a trained machine learning model

- wherein the machine learning model is based on a machine learning method such as a logistic regression, LDA, Random Forest analysis, decision tree analysis, XGBoost analysis, decision tree analysis or SVM using the expression data,
- wherein the machine learning model is trained based on a training cohort of patients with lung cancer subtypes NSCLC and of patients with SCLC and corresponding diagnostic, prognostic and/or predictive conclusions in the context of NSCLC disease or SCLC disease; and

(c) deriving a diagnostic, prognostic and/or predictive conclusion output with respect to the detection of the lung cancer subtypes NSCLC or SCLC in the subject.

12. A computer-implemented method for providing a trained machine learning model for the analysis of a sample, comprising the steps:

(a) obtaining data on the levels of expression of a group of biomarkers comprising CYFRA 21-1, SCC, NSE, TIMP-1, and Her2/Neu from a cohort of patients with lung cancer subtype NSCLC and of patients with cancer subtype SCLC from a data storage;

(b) performing a machine learning method such as a logistic regression, LDA, Random Forest analysis, XGBoost analysis, decision tree analysis or SVM using the levels of expression of CYFRA 21-1, SCC, NSE, TIMP-1, and Her2/Neu of the cohorts of step (a);

(c) training a machine learning model with the result obtained in step (b) and with diagnostic, prognostic and/or predictive conclusions in the context of lung cancer subtypes NSCLC or SCLC corresponding to said expression, thereby obtaining a trained machine learning model.

13. The computer-implemented method of any one of claims 7 to 12, wherein the training of the machine learning model additionally includes the inputting of data of the cohorts concerning

(i) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or

(ii) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or

(iii) detectable molecular changes, preferably on the level of the genome, epigenome, fragmentome, transcriptome, metabolome, proteome, glycome or lipidome and/or laboratory tests.

14. A method of detecting and assessing a lung cancer disease in a subject, comprising the steps of:

(i) performing the method of claim 7;

(ii) introducing the results obtained in step (i) and, optionally, introducing (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests into a first machine learning model as obtained in claim 8;

(iii) determining with said first machine learning model a likelihood for the presence of a lung cancer disease in the subject;

(iv) performing in a subject with a high likelihood as determined in step (iii) the method of claim 9;

(v) introducing the results obtained in step (iv) and, optionally, introducing (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests into a second machine learning model as obtained in claim 10;

(vi) determining with said second machine learning model a likelihood for the presence of a lung cancer disease vs. a non-cancerous lung disease in the subject;

(vii) performing in a subject with a high likelihood of lung cancer disease as determined in step (vi) the method of claim 11;

(viii) introducing the results obtained in step (vii) and, optionally, introducing (1) imaging-based marker, such as vascular convergence; pleural indentation; air bronchogram; calcification type; cavitation; endobronchial origin; perifissural location; subpleural location; morphology; spiculation, lobulation, associated cystic airspace, bubble-like lucencies in a nodule; and/or (2) non-imaging-based data, such as data on age, sex, smoking status (former or current smoker), smoking intensity, smoking duration, duration of quitting smoking, ethnicity, personal history of cancer, familial cancer predisposition, Body Mass Index, Education, or other presence of lung diseases like COPD; and/or (3) detectable molecular changes, preferably on the level of the genome, epigenome, fragmentome, transcriptome, metabolome, proteome, glycome or lipidome and/or laboratory tests into a third machine learning model as obtained in claim 12; and

(ix) determining with said third machine learning model the likelihood for the presence of a lung cancer subtype NSCLC or SCLC.

15. The method of any one of claims 4 to 13, wherein said sample is a body fluid sample, preferably a blood sample such as a serum or plasma sample, an exhaled breath condensate, a breath sample, a saliva sample, or a bronchial brushing sample.

FIG 1

FIG 2

FIG 3

□ ～17    ■ ～18

100% ─ ～18
80%
60% ─ ～17
40%
20%
0%
20    21
～18
～17

FIG 4

2.00 ─ 19 10 6
1.75
1.50
1.25
1.00
0.75
0.50
0.25
0.00
-4  -3  -2  -1  0  1  2  3  4
2

## FIG 5

## FIG 6

FIG 7

EP 4 692 801 A1

# FIG 8

31

FIG 9

FIG 10

Europäisches
Patentamt

European
Patent Office

Office européen
des brevets

# EUROPEAN SEARCH REPORT

**Application Number**

EP 24 19 3243

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HIRALES CASILLAS CARLOS ENRIQUE ET AL: "Current status of circulating protein biomarkers to aid the early detection of lung cancer", FUTURE ONCOLOGY, vol. 10, no. 8, 1 June 2014 (2014-06-01), pages 1501-1513, XP093104595, GB ISSN: 1479-6694, DOI: 10.2217/fon.14.21 * the whole document * * in particular: * * abstract * * table 1 * * section "Circulating protein biomarkers for early detection of lung cancer"; page 1504 - page 1507 * | 1,3,4,7, 8,11-15 | INV. G01N33/574 |
| X | "43th ISOBM Annual Congress of International Society of Oncology and BioMarkers, September 1-6, 2016 Chicago, USA", TUMOR BIOLOGY, KARGER, BASEL, CH, vol. 37, no. 1, 20 August 2016 (2016-08-20), pages 1-45, XP036039526, ISSN: 1010-4283, DOI: 10.1007/S13277-016-5287-4 [retrieved on 2016-08-20] | 6 | |
| Y | * Abstract 171; page S30 * | 1,3,4,7, 8,11-15 | TECHNICAL FIELDS SEARCHED (IPC) G01N |

-/--

The present search report has been drawn up for all claims

| Place of search | Date of completion of the search | Examiner |
|---|---|---|
| The Hague | 9 January 2025 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 19 3243

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 11 105 806 B2 (CELERA CORP [US]) 31 August 2021 (2021-08-31) * the whole document * * in particular: * * tables 1,3,9,10 * * claims 1,2,17,21 * * column 3, line 5 - line 17 * * column 3, line 29 - line 39 * * column 4, line 56 - line 67 * * column 12, line 63 - column 13, line 13 * * column 22, line 49 - column 23, line 26 * * column 92, line 50 - column 93, line 30 * | 1,3,4,7, 8,11-15 | |
| Y | WO 2021/215986 A1 (SJOEBLOM TOBIAS [SE]; EKSTROEM JOAKIM [SE]) 28 October 2021 (2021-10-28) * the whole document * * in particular: * * claims 1-6,24,25 * | 1,3,4,7, 8,11-15 | |
| Y | VISSER ESTHER ET AL: "Liquid biopsy-based decision support algorithms for diagnosis and subtyping of lung cancer", LUNG CANCER., vol. 178, 1 April 2023 (2023-04-01), pages 28-36, XP093236684, NL ISSN: 0169-5002, DOI: 10.1016/j.lungcan.2023.01.014 * the whole document * * in particular: * * abstract * * section "2. Materials and methods"; page 29 - page 30 * * figures 2-4 * | 11-15 | |

**TECHNICAL FIELDS SEARCHED (IPC)**

The present search report has been drawn up for all claims

| Place of search | Date of completion of the search | Examiner |
|---|---|---|
| The Hague | 9 January 2025 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................
& : member of the same patent family, corresponding document

EPO FORM 1503 03.82 (P04C01)

**page 2 of 2**

Europäisches
Patentamt

European
Patent Office

Office européen
des brevets

**Application Number**

EP 24 19 3243

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1, 3, 4, 6-8, 11, 12, 14(completely); 13, 15(partially)

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

Application Number

EP 24 19 3243

The Search Division considers that the present European patent application does not comply with the requirements of unity of invention and relates to several inventions or groups of inventions, namely:

1. claims: 1, 3, 4, 6-8, 11, 12, 14(completely); 13, 15(partially)

   Methods and products for detecting a lung cancer disease or for discriminating lung cancer subtypes Non-small-cell lung cancer (NSCLC) and small-cell lung cancer (SCLC)
   ---

2. claims: 2, 5, 9, 10(completely); 13, 15(partially)

   Methods and products for discriminating a non-cancerous lung disease from a lung cancer disease in a subject
   ---

## ANNEX TO THE EUROPEAN SEARCH REPORT
## ON EUROPEAN PATENT APPLICATION NO.

EP 24 19 3243

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2025

| Patent document cited in search report | Publication date | Patent family member(s) | Publication date |
|---|---|---|---|
| US 11105806 B2 | 31-08-2021 | CA 2705486 A1 | 28-05-2009 |
| | | EP 2227556 A2 | 15-09-2010 |
| | | EP 2728017 A1 | 07-05-2014 |
| | | EP 3115469 A1 | 11-01-2017 |
| | | JP 5580205 B2 | 27-08-2014 |
| | | JP 5812545 B2 | 17-11-2015 |
| | | JP 2011527414 A | 27-10-2011 |
| | | JP 2014089217 A | 15-05-2014 |
| | | JP 2015143716 A | 06-08-2015 |
| | | JP 2017181525 A | 05-10-2017 |
| | | US 2009176228 A1 | 09-07-2009 |
| | | US 2012076725 A1 | 29-03-2012 |
| | | US 2015037820 A1 | 05-02-2015 |
| | | US 2017153238 A1 | 01-06-2017 |
| | | US 2020064347 A1 | 27-02-2020 |
| | | WO 2009067546 A2 | 28-05-2009 |
| WO 2021215986 A1 | 28-10-2021 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DIAMANDIS**. *BMC Med*, 2012, vol. 10, 87
- **YANG et al.** *Sci Rep*, 2021, vol. 11 (1), 19044
- **VISSER et al.** *Lung Cancer*, 2023, vol. 178, 28-36
- **YOUDEN**. *Cancer*, 1950, vol. 3 (1), 32-35
- Linear Discriminant Analysis. **PETROS XANTHO-POULOS** ; **PANOS M. PARDALOS** ; **THEODORE B. TRAFALIS**. Robust Data Mining. Springer, 2012, 27-33
- **L. BREIMAN**. *Machine Learning*, 2001, vol. 45, 5-32
- **CHEN et al.** *Proceedings of the 22nd ACM SIGKDD International Conference on Knowledge Discovery and Data Mining*, 2016, 785-794
- **JIAO et al.** *J. Phys.: Conf. Ser*, 2020, vol. 1651, 012083
- **C. CORTES** ; **V. VAPNIK**. *Machine Learning*, 1995, vol. 20, 273-29